# EUROPÄISCHE PATENTSCHRIFT

(11) **EP 3 266 410 B1**
(45) Veröffentlichungstag und Bekanntmachung des Hinweises auf die Patenterteilung: **01.01.2020**
(21) Anmeldenummer: 16177709.9
(22) Anmeldetag: 04.07.2016
(51) Int. Cl.: A61C 17/20, A61C 17/22, A61C 1/00, A61C 1/08, A61B 90/30

(54) **MEDIZINISCHE ODER DENTALE BEHANDLUNGSVORRICHTUNG UND WERKZEUG FÜR EINE DERARTIGE BEHANDLUNGSVORRICHTUNG**
MEDICAL OR DENTAL TREATMENT DEVICE AND TOOL FOR SUCH A TREATMENT DEVICE
DISPOSITIF DE TRAITEMENT DENTAIRE OU MÉDICAL ET OUTIL POUR UN TEL DISPOSITIF DE TRAITEMENT

(43) Veröffentlichungstag der Anmeldung: 10.01.2018
(60) Teilanmeldung: 19209625.3
(73) Patentinhaber: W & H Dentalwerk Bürmoos GmbH, 5111 Bürmoos (AT)
(72) Erfinder: BRANDSTÄTTER, Andreas, 5120 St. Pantaleon (AT); PRUCKNER, Christian, 1190 Wien (AT); AUER, Theresa, 5110 Oberndorf (AT); EIBL, Johann, 5230 Mattighofen (AT)
(74) Vertreter: Benda, Ralf

(56) Entgegenhaltungen:
- EP-A1- 2 184 028
- WO-A1-2014/140448
- DE-A1- 3 201 800
- DE-A1-102006 057 338
- DE-C2- 3 708 801
- US-A1- 2008 293 008
- US-A1- 2011 195 377

## Beschreibung

Die vorliegende Erfindung betrifft eine medizinische oder dentale Behandlungsvorrichtung, die mit mehreren unterschiedlichen Werkzeugen verbindbar ist, wobei die medizinische oder dentale Behandlungsvorrichtung eine Auswertevorrichtung zur Erkennung, ob eines der mehreren Werkzeuge mit der Behandlungsvorrichtung verbunden ist oder welches der mehreren Werkzeuge mit der Behandlungsvorrichtung verbunden ist, aufweist. Die Erfindung betrifft auch ein entsprechendes Verfahren und Werkzeuge, die mit einer derartigen medizinischen oder dentalen Behandlungsvorrichtung unterscheidbar sind.

Aus der Patentanmeldung US 2010/109644 A1 ist eine derartige medizinische oder dentale Behandlungsvorrichtung bekannt. Diese Behandlungsvorrichtung, die eine Auswertevorrichtung zur Erkennung, ob eines der mehreren Werkzeuge mit der Behandlungsvorrichtung verbunden ist oder welches der mehreren Werkzeuge mit der Behandlungsvorrichtung verbunden ist, umfasst, funktioniert hervorragend und wird von den Anwender sehr geschätzt.

Der vorliegenden Erfindung liegt nun die Aufgabe zugrunde, eine Behandlungsvorrichtung zu schaffen, deren Auswertevorrichtung derart weiterentwickelt ist, dass eine größere Anzahl von Werkzeugen oder Werkzeuggruppen unterscheidbar ist. Demgemäß ist es auch ein Ziel der vorliegenden Erfindung ein Verfahren und ein Werkzeug zu schaffen, mit dem eine größere Anzahl von Werkzeugen oder Werkzeuggruppen unterscheidbar ist.

Diese Aufgabe wird gemäß der vorliegenden Erfindung durch eine medizinische oder dentale Behandlungsvorrichtung mit den Merkmalen des Anspruchs 1 und ein Verfahren zur Erkennung ob ein Werkzeug mit einer Behandlungsvorrichtung oder welches von mehreren Werkzeugen mit der Behandlungsvorrichtung verbunden ist / sind mit den Merkmalen des Anspruchs 13 gelöst. Besonders vorteilhafte Ausführungsformen sind in den jeweiligen Unteransprüchen angeführt.

Die erfindungsgemäße medizinische oder dentale Behandlungsvorrichtung, die mit mehreren unterschiedlichen Werkzeugen verbindbar ist, umfasst eine Werkzeughalterung zum Verbinden der Behandlungsvorrichtung mit einem Werkzeug, zumindest eine Messspule, die über eine elektrische Leitung mit einer elektrischen Energieversorgungsvorrichtung verbunden ist und von der elektrischen Energieversorgungsvorrichtung mit einer periodischen, insbesondere sinusförmigen, elektrischen Energie (Wechselstrom) versorgbar ist, und eine mit der zumindest einen Messspule elektrisch verbundene Auswertevorrichtung zur Erkennung ob ein Werkzeug mit der Behandlungsvorrichtung verbunden ist oder welches von mehreren Werkzeugen mit der Behandlungsvorrichtung verbunden ist. Aufgrund einer elektromagnetischen, insbesondere induktiven, Kopplung zwischen dem in der Werkzeughalterung aufgenommenen Werkzeug und der mit der periodischen, insbesondere sinusförmigen, Energie versorgten zumindest einen Messspule ist ein für jedes Werkzeug spezifisches, durch die Auswertevorrichtung dem jeweiligen Werkzeug zuordenbares periodisches, insbesondere sinusförmiges, Messsignal generierbar.

Erfindungsgemäß bestimmt die Auswertevorrichtung zur Erkennung, ob ein Werkzeug mit der Behandlungsvorrichtung verbunden ist oder welches von mehreren Werkzeugen mit der Behandlungsvorrichtung verbunden ist, den Phasenversatz oder die Phasenverschiebung der elektrischen Spannung und des elektrischen Stroms des periodischen, insbesondere sinusförmigen, Messsignals.

Vorzugsweise umfasst die medizinische oder dentale Behandlungsvorrichtung zumindest ein Werkzeug sowie die im Vorstehenden genannten Komponenten Werkzeughalterung, zumindest eine Messspule, und eine mit der zumindest einen Messspule elektrisch verbundene Auswertevorrichtung zur Erkennung ob ein Werkzeug mit der Behandlungsvorrichtung verbunden ist oder welches von mehreren Werkzeugen mit der Behandlungsvorrichtung verbunden ist. Das zumindest eine Werkzeug umfasst einen Werkzeugschaft und ein mit dem Werkzeugschaft verbundenes Arbeitsende zum Bearbeiten einer Behandlungsstelle, wobei der Werkzeugschaft ein elektrisch leitfähiges Identifizierungselement aufweist, in dem durch die elektromagnetische, insbesondere induktive, Kopplung zwischen dem in der Werkzeughalterung aufgenommenen Werkzeug und der mit der periodischen, insbesondere sinusförmigen, Energie versorgten zumindest einen Messspule ein (induzierter) elektrischer Wechselstrom und / oder elektrische Wirbelströme induzierbar ist / sind, der / die zur Generierung des dem jeweiligen Werkzeug zuordenbaren periodischen, insbesondere sinusförmigen, Messsignals auf die in der zumindest einen Messspule fließende periodische, insbesondere sinusförmige, elektrische Energie, insbesondere über Gegeninduktivität und / oder Ohmsche Verluste, (rück)wirkt / (rück)wirken. Das Wirken auf die in der zumindest einen Messspule fließende periodische, insbesondere sinusförmige, elektrische Energie erfolgt insbesondere indirekt durch die sich ändernden Magnetfelder und / oder elektrischen Felder des elektrischen Wechselstroms und / oder der elektrischen Wirbelströme.

Wie im Folgenden noch im Detail beschrieben basiert die Erkennung oder Unterscheidung mehrerer Werkzeug, die mit der medizinischen oder dentalen Behandlungsvorrichtung verbindbar sind, insbesondere darauf, dass jedes Werkzeug ein spezifisches elektrisch leitfähiges Identifizierungselement, d.h. ein elektrisch leitfähiges Identifizierungselement mit zumindest einer individuellen Eigenschaft oder einem individuellen Parameter, aufweist. Diese individuelle Eigenschaft oder dieser individuelle Parameter bewirkt eine für dieses Identifizierungselement spezifische Wirkung oder Rückwirkung auf die in der zumindest einen Messspule fließende periodische, insbesondere sinusförmige, elektrische Energie, wodurch das dem jeweiligen Werkzeug eindeutig zuordenbare oder spezifische, periodische, insbesondere sinusförmige, Messsignal generierbar ist. Die Auswertevorrichtung oder ein Komparator ist insbesondere dazu ausgebildet, dieses empfangene spezifische, periodische, insbesondere sinusförmige, Messsignal mit vorbestimmten Vergleichswerten, die zum Beispiel in einem Speicher der Auswertevorrichtung gespeichert sind, zu vergleichen und das in der Werkzeughalterung aufgenommenen Werkzeug zu erkennen.

Vorzugsweise bestimmt die Auswertevorrichtung zur Erkennung, ob ein Werkzeug mit der Behandlungsvorrichtung verbunden ist oder welches von mehreren Werkzeugen mit der Behandlungsvorrichtung verbunden ist, zusätzlich zur Phasenverschiebung des periodischen, insbesondere sinusförmigen, Messsignals die Amplitude, insbesondere die Amplitudenextremwerte, der elektrischen Spannung des periodischen, insbesondere sinusförmigen, Messsignals. Dies erhöht die Anzahl der voneinander unterscheidbaren Werkzeuge oder Werkzeuggruppe weiter.

Das Bestimmen der Phasenverschiebung der elektrischen Spannung und des elektrischen Stroms des periodischen, insbesondere sinusförmigen, Messsignals bewirkt, dass eine größere Anzahl von Werkzeugen oder Werkzeuggruppen unterscheidbar ist. Besonders bevorzugt wird durch das Vorsehen eines Werkzeugs mit einem elektrisch leitfähigem Identifizierungsmatrial, insbesondere in einer medizinischen oder dentalen Behandlungsvorrichtung, die Anzahl der voneinander unterscheidbaren Werkzeuge oder Werkzeuggruppe noch erheblich gesteigert. Ausgehend von der aus dem Stand der Technik bekannten Anzahl der voneinander unterscheidbaren Werkzeuge oder Werkzeuggruppen konnte in Versuchen bei Anwendung der Erfindung eine Steigerung der Anzahl der zuverlässig voneinander unterscheidbaren Werkzeuge oder Werkzeuggruppen um etwa 2/3, bei einer zusätzlichen Anwendung eines Werkzeugs mit einem elektrisch leitfähigen Identifizierungselement eine Steigerung um das etwa 3 - 6fache festgestellt werden.

Vorzugsweise bilden zumindest die Messspule und die Auswertevorrichtung, insbesondere auch die elektrische Energieversorgungsvorrichtung, einen elektrischen Messkreis zur Erkennung ob ein Werkzeug mit der Behandlungsvorrichtung verbunden ist oder welches von mehreren Werkzeugen mit der Behandlungsvorrichtung verbunden ist. Vorzugsweise umfasst der elektrische Messkreis weitere Komponenten, zum Beispiel Filter oder Verstärker, insbesondere für das periodische, insbesondere sinusförmige, Messsignal, einen Mikrokontroller, einen Komparator zum Vergleichen des von der Auswertevorrichtung empfangenen periodischen, insbesondere sinusförmigen, Messsignals oder eines davon abgeleiteten Signals mit vorgegebenen Vergleichswerten, um dem periodischen, insbesondere sinusförmigen, Messsignal ein Werkzeug, insbesondere das in der Werkzeughalterung aufgenommene Werkzeug, zuzuordnen und / oder eine Anzeige, um dem Anwender den Namen des oder Betriebsdaten für das Werkzeug, insbesondere das in der Werkzeughalterung aufgenommene Werkzeug, anzuzeigen.

Vorzugsweise ist die Auswertevorrichtung ausgebildet, das in oder an der Messspule generierte periodische, insbesondere sinusförmige, Messsignal zu empfangen und auszuwerten, um zu erkennen, ob ein Werkzeug mit der Behandlungsvorrichtung verbunden ist oder welches von mehreren Werkzeugen mit der Behandlungsvorrichtung verbunden ist. Besonders bevorzugt umfasst die Auswertevorrichtung oder ein Mikrokontroller der Behandlungsvorrichtung ein Computerprogramm mit Programmcodemitteln zur Durchführung eines Verfahrens zur Erkennung ob ein Werkzeug mit einer Behandlungsvorrichtung verbunden ist oder welches von mehreren Werkzeugen mit der Behandlungsvorrichtung verbunden ist, so wie es im Folgende beschrieben ist. Die Erfindung oder das Computerprogramm ist bevorzugt in Software implementiert.

Vorzugsweise umfasst die medizinische oder dentale Behandlungsvorrichtung ein medizinisches oder dentales Handstück oder Winkelstück und eine Steuervorrichtung, die insbesondere beabstandet voneinander ausgebildet und / oder durch einen Schlauch miteinander verbunden sind. Vorzugsweise ist zumindest ein Teil der Auswertevorrichtung in der Steuervorrichtung vorgesehen und / oder als Teil der Steuervorrichtung ausgebildet. Vorzugsweise sind an der Steuervorrichtung weitere Komponenten vorgesehen, zum Beispiel Stellelemente zum Auswählen und / oder Einstellen von Betriebsparametern und / oder Betriebsmitteln und / oder Betriebsmittelmengen durch den Anwender, eine Anzeige und / oder eine Flüssigkeitsversorgungseinheit, die ausgebildet ist, eine Behandlungsflüssigkeit, insbesondere zur Abgabe auf eine Behandlungsstelle, an das Handstück oder Winkelstück zu fördern. Vorzugsweise ist die, insbesondere kraft- oder formschlüssige, Werkzeughalterung an oder in dem Handstück oder Winkelstück angeordnet. Vorzugsweise ist die medizinische oder dentale Behandlungsvorrichtung als Table-Top-Gerät ausgebildet.

Vorzugsweise umfasst die medizinische oder dentale Behandlungsvorrichtung eine Antriebseinheit, die ausgebildet ist, das mit der Behandlungsvorrichtung verbundene Werkzeug in ein Antriebsbewegung, zum Beispiel in ein rotierende und / oder schwingende und / oder oszillierende und / oder vibrierende Antriebsbewegung zu versetzen. Vorzugsweise ist die Antriebseinheit in dem Handstück oder Winkelstück oder anschließend daran angeordnet. Besonders bevorzugt umfasst die Antriebseinheit einen Luftmotor oder einen Elektromotor oder einen pneumatischen, piezoelektrischen oder magnetostriktiven Schwingantrieb.

Vorzugsweise umfasst die medizinische oder dentale Behandlungsvorrichtung eine Vorrichtung zum Entfernen von Zahnstein, Plaque, Biofilm und / oder Verfärbungen auf Zähnen mit einem pneumatischen, piezoelektrischen oder magnetostriktiven Schall-/oder Ultraschall-Schwingantrieb, die oftmals als Scale-Vorrichtung bezeichnet ist. Alternativ umfasst die medizinische oder dentale Behandlungsvorrichtung andere Vorrichtungen für die Restauration, Prothetik, Endodontie, Implantologie, oder andere Anwendungen, insbesondere mit einem in eine Dreh- oder Rotationsbewegung versetzbaren Werkzeug.

Demgemäß ist das Werkzeug vorzugsweise als rotierendes Werkzeug, als oszillierendes Werkzeug, als in Vibration versetzbares Werkzeug, als mit Schall oder Ultraschall antreibbares Werkzeug oder als Zahnsteinentfernungs-Werkzeug ausgebildet.

Vorzugsweise ist die Messspule in dem Handstück oder Winkelstück, insbesondere an der oder anschließend an die Werkzeughalterung angeordnet. Insbesondere ist die Messspule derart ausgebildet und / oder in dem Handstück oder Winkelstück angeordnet, dass das elektrisch leitfähige Identifizierungselement in der Messspule positionierbar ist, insbesondere wenn das Werkzeug in der Werkzeughalterung aufgenommen oder eingespannt ist.

Die elektrische Energieversorgungsvorrichtung ist vorzugsweise ausgebildet, periodische, insbesondere sinusförmige, elektrischen Energie (Wechselstrom) zu generieren und die zumindest eine Messspule mit periodischer, insbesondere sinusförmiger, elektrischer Energie (Wechselstrom) zu versorgen. Die elektrische Energieversorgungsvorrichtung kann wahlweise in der medizinischen oder dentalen Behandlungsvorrichtung, insbesondere in der Steuervorrichtung, oder außerhalb der medizinische oder dentale Behandlungsvorrichtung vorgesehen sein. Eine Anordnung der elektrischen Energieversorgungsvorrichtung in der Behandlungsvorrichtung, insbesondere in einem Mikrokontroller oder als Teil einer Mikrokontrollereinheit, erleichtert in vorteilhafter Weise die Bestimmung der Phasenverschiebung der elektrischen Spannung und des elektrischen Stroms des periodischen, insbesondere sinusförmigen, Messsignals, da der Auswerteeinheit der für die Bestimmung der Phasenverschiebung benötigte Nullpunkt der periodischen, insbesondere sinusförmigen, elektrischen Energie bekannt ist oder durch die Auswerteeinheit einfach ermittelbar ist. Besonders bevorzugt ist die elektrische Energieversorgungsvorrichtung als Teil eines Mikrokontrollers oder einer Mikrokontrollereinheit der Behandlungsvorrichtung ausgebildet und / oder durch einen Mikrokontroller der Behandlungsvorrichtung mittels Software gesteuert. Besonders bevorzugt umfasst die elektrische Energieversorgungsvorrichtung einen Signalgenerator.

Die elektromagnetische, insbesondere induktive, Kopplung zwischen dem in der Werkzeughalterung aufgenommenen Werkzeug und der mit der periodischen, insbesondere sinusförmigen, Energie versorgten zumindest einen Messspule zur Erzeugung eines für jedes Werkzeug spezifischen, durch die Auswertevorrichtung dem jeweiligen Werkzeug zuordenbaren periodischen, insbesondere sinusförmigen, Messsignals umfasst zum Beispiel eine Beeinflussung oder Veränderung der Induktivität der zumindest einen Messspule, die zum Beispiel durch die weichmagnetischen Eigenschaften des Werkzeugs und / oder des elektrisch leitfähigen Identifizierungselements verursacht ist. Die elektromagnetische, insbesondere induktive, Kopplung umfasst zusätzlich oder alternativ auch die Generierung des für jedes Werkzeug spezifischen periodischen, insbesondere sinusförmigen, Messsignals durch Gegeninduktivität und / oder Ohmsche Verluste, die zum Beispiel durch in dem Werkzeug und / oder dem elektrisch leitfähigen Identifizierungselement induzierten Wechselstrom und / oder Wirbelströme und den daraus resultierenden sich ändernden Magnetfeldern oder elektrischen Feldern, bewirkt wird.

Die von der elektrischen Energieversorgungsvorrichtung zur Verfügung gestellte periodische, insbesondere sinusförmige, elektrische Energie bildet insbesondere das Ausgangssignal oder die Basis des periodischen, insbesondere sinusförmigen, Messsignals. Aufgrund der elektromagnetischen, insbesondere induktiven, Kopplung zwischen dem in der Werkzeughalterung aufgenommenen Werkzeug und der zumindest einen Messspule wird zumindest ein Parameter der periodischen elektrischen Energie, zum Beispiel die elektrische Spannung, die elektrische Stromstärke oder die Amplitude der elektrischen Spannung, beeinflusst oder verändert und dadurch das für jedes der mehreren Werkzeuge spezifische, periodische, insbesondere sinusförmige, Messsignal generiert.

Das zumindest eine Werkzeug weist vorzugsweise einen Werkzeugschaft und ein mit dem Werkzeugschaft verbundenes Arbeitsende zum Bearbeiten einer Behandlungsstelle auf. Der Werkzeugschaft ist vorzugsweise zylindrisch geformt. An dem Werkzeugschaft ist vorzugsweise zumindest eine geometrische Struktur zum Verbinden mit der Werkzeughalterung und / oder zum Eingriff eines Halteelements der Werkzeughalterung vorgesehen, zum Beispiel ein Gewinde, ein Rücksprung oder eine Vertiefung. Das Arbeitsende des Werkzeugs ist vorzugsweise abrasiv ausgebildet und umfasst zum Beispiel zumindest eine Schneidkante und / oder abrasive Partikel.

Vorzugsweise weist das zumindest eine elektrisch leitfähige Identifizierungselement des Werkzeugschafts ein Material mit einer hohen elektrischen Leitfähigkeit auf, insbesondere ein metallisches Material, zum Beispiel Kupfer, Gold, Silber, Zinn, Wismut oder eine metallische Legierung, vorzugsweise mit zumindest einem der vorgenannten Metalle, zum Beispiel Messing, Bronze oder Neusilber.

Vorzugsweise ist das zumindest eine elektrisch leitfähige Identifizierungselement auf einem Grundmaterial des Werkzeugschafts vorgesehen, wobei das zumindest eine elektrisch leitfähige Identifizierungselement und das Grundmaterial unterschiedliche Materialien aufweisen. Vorzugsweise umfasst das Grundmaterial des Werkzeugschafts ein Kunststoff oder ein metallisches Material, zum Beispiel Stahl. Alternativ ist es auch denkbar, dass das zumindest eine elektrisch leitfähige Identifizierungselement und der Werkzeugschaft aus demselben Material und / oder einteilig hergestellt sind.

Vorzugsweise weist das zumindest eine elektrisch leitfähige Identifizierungselement einen Draht, eine Folie, Hülse, Wicklung oder Spule auf oder ist als solche ausgebildet. Vorzugsweise ist das zumindest eine elektrisch leitfähige Identifizierungselement, insbesondere der Draht, die Folie, Hülse, Wicklung oder Spule, durch Kleben, Pressen, Schrumpfen, Verschrauben oder Schweißen an dem Werkzeug, bevorzugt unlösbar, befestigt.

Alternativ weist das zumindest eine elektrisch leitfähige Identifizierungselement eine Beschichtung auf oder ist als solche ausgebildet. Vorzugsweise ist die Beschichtung durch chemische oder galvanische Abscheidung des Materials des zumindest einen elektrisch leitfähigen Identifizierungselements auf dem Werkzeug hergestellt. Besonders bevorzugt umfasst das zumindest eine elektrisch leitfähige Identifizierungselement eine Wicklung oder Spule, die durch Beschichtung des Werkzeugs mit dem Material des zumindest einen elektrisch leitfähige Identifizierungselements und anschließender mechanischer, chemischer oder Laser-Bearbeitung der Beschichtung zum Entfernen eines Teils der Beschichtung, um dadurch die Wicklung oder Spule zu erhalten, hergestellt ist.

Alternativ weist das zumindest eine elektrisch leitfähige Identifizierungselement eine an dem Werkzeug zum Beispiel durch Kleben, Pressen, Schrumpfen oder Verschrauben befestigbare Kunststoffhülse mit einer darauf vorgesehenen metallischen Beschichtung auf. Damit ist es in vorteilhafter Weise möglich, das gleiche, insbesondere metallische, Material für das Grundmaterial des Werkzeugs oder des Werkzeugschafts und für das zumindest eine elektrisch leitfähige Identifizierungselement zu verwenden.

Vorzugsweise ist das zumindest eine elektrisch leitfähige Identifizierungselement, insbesondere wenn dieses als Beschichtung oder Folie ausgebildet ist, durch eine zusätzlich auf dem Werkzeug vorgesehene oder abgeschieden Schicht bedeckt. Das zumindest eine elektrisch leitfähige Identifizierungselement ist somit insbesondere zwischen dieser zusätzlichen Schicht und dem Grundmaterial des Werkzeugschafts angeordnet. Die zusätzliche Schicht bildet bevorzugt eine Schutzschicht mit im Vergleich zum Material des zumindest einen elektrisch leitfähigen Identifizierungselements erhöhter Beständigkeit, zum Beispiel gegenüber Korrosion oder mechanischer Belastung, eine Passivierungsschicht des Materials des zumindest einen elektrisch leitfähigen Identifizierungselements, zum Beispiel in Form einer Oxid-Schicht, oder eine optische Barriere, um das Vorhandensein des zumindest einen elektrisch leitfähigen Identifizierungselements zu verbergen.

Vorzugsweise bildet das zumindest eine elektrisch leitfähige Identifizierungselement einen den Werkzeugschaft umgebenden geschlossenen elektrischen Stromkreis, in dem ein (regelmäßig, periodisch seine Polung ändernder) elektrischer Wechselstrom, insbesondere um den Werkzeugschaft oder um eine Längsachse des Werkzeugschafts, fließen kann. Vorzugsweise fließt der elektrische Wechselstrom als Ringstrom in dem elektrisch leitfähigen Identifizierungselement um den Werkzeugschaft oder um eine Längsachse des Werkzeugschafts. Vorzugsweise ist das elektrisch leitfähige Identifizierungselement als in sich geschlossene(r) (d.h. keine Enden aufweisende) Spule, Wicklung, Draht, Folie oder Beschichtung ausgebildet, um den geschlossenen elektrischen Stromkreis zu bilden. Um unterschiedliche Werkzeuge zu erkennen, reicht überraschenderweise für die Bildung des geschlossenen elektrischen Stromkreises eine einzige (den Werkzeugschaft mit 360° umgebende) Windung oder Wicklung der Spule, Wicklung, Draht, Folie oder Beschichtung aus. Besonders bevorzugt und überraschend kann diese einzige Windung oder Wicklung auch hülsenförmig oder als Hülse ausgebildet sein, so dass das zumindest eine elektrisch leitfähige Identifizierungselement insbesondere auch eine zylindrische Hülse aufweisen kann, um einen den Werkzeugschaft umgebenden geschlossenen elektrischen Stromkreis zu bilden.

Ein derartig durch das zumindest eine elektrisch leitfähige Identifizierungselement gebildeter geschlossener elektrischer Stromkreis, insbesondere der darin fließende (induzierte) Wechselstrom oder Ringstrom, bewirken durch die elektromagnetische, insbesondere induktive Kopplung, ein besonders wirkungsvolles Rückwirken auf die in der zumindest einen Messspule fließende periodische, insbesondere sinusförmige, elektrische Energie, insbesondere durch Gegeninduktivität, zur Generierung des dem jeweiligen Werkzeug zuordenbaren periodischen, insbesondere sinusförmigen, Messsignals.

Alternativ weist das zumindest eine elektrisch leitfähige Identifizierungselement zwei elektrisch voneinander getrennte Enden auf. Somit bildet das zumindest eine elektrisch leitfähige Identifizierungselement keinen geschlossenen elektrischen Stromkreis. Demgemäß ist in einem derartigen Identifizierungselement kein (regelmäßig, periodisch seine Polung ändernder) elektrische Wechselstrom oder um den Werkzeugschaft oder um eine Längsachse des Werkzeugschafts fließender Ringstrom induzierbar, sondern es sind ausschließlich elektrische Wirbelström darin induzierbar. Überraschenderweise wurde festgestellt, dass diese elektrischen Wirbelström ebenfalls geeignet sind, die gewünschte Erkennung oder Unterscheidung von mehreren unterschiedlichen Werkzeugen durch Veränderung oder Beeinflussung der in der zumindest einen Messspule fließenden periodischen, insbesondere sinusförmigen, elektrischen Energie, insbesondere durch Ohmsche Verluste, zu bewirken.

Das zumindest eine elektrisch leitfähige Identifizierungselement mit zwei elektrisch voneinander getrennte Enden umfasst zum Beispiel eine geschlitzte Hülse, eine Spule, Wicklung oder einen Draht mit zwei freien oder elektrisch unverbundenen Enden, eine entsprechend der Hülse geschlitzte Beschichtung oder eine der Wicklung oder dem Draht entsprechende Beschichtung mit zwei freien oder elektrisch unverbundenen Enden. Die zwei voneinander elektrisch getrennten Enden sind insbesondere durch einen zwischen diesen beiden Enden vorgesehenen Abstand oder Schlitz gebildet.

Vorzugsweise umfasst die medizinische oder dentale Behandlungsvorrichtung mehrere unterschiedliche Werkzeuge, von denen jedes einen Werkzeugschaft mit zumindest einem elektrisch leitfähigen Identifizierungselement aufweist. Vorzugsweise sind diese mehreren unterschiedlichen Werkzeuge in einem Set von Werkzeugen zusammengefasst. Wie im Vorstehenden bereits beschrieben, basiert insbesondere die Erkennung oder Unterscheidung dieser mehreren Werkzeug, die mit der medizinischen oder dentalen Behandlungsvorrichtung verbindbar sind, darauf, dass jedes Werkzeug ein spezifisches elektrisch leitfähiges Identifizierungselement, d.h. ein elektrisch leitfähiges Identifizierungselement mit zumindest einer individuellen Eigenschaft oder einem individuellen Parameter, aufweist.

Vorzugsweise unterscheiden sich die elektrisch leitfähigen Identifizierungselemente der Werkzeuge, insbesondere des Werkzeugsets, durch zumindest einen der folgenden Parameter:
- die axialen Längen der elektrisch leitfähigen Identifizierungselemente in Bezug auf eine Längsachse des Werkzeugschafts; vorzugsweise beträgt die axiale Länge eines elektrisch leitfähigen Identifizierungselements zwischen 0,5 mm bis 10 mm; vorzugsweise unterscheiden sich die axialen Längen mehrerer elektrisch leitfähiger Identifizierungselemente, insbesondere eines Sets von Werkzeugen, (jeweils) um 0,5 mm, 1 mm, 1,5 mm, 2 mm, 2,5 mm, um 5 mm oder um 10 mm;
- die Querschnittsflächen oder Drahtdurchmesser der elektrisch leitfähigen Identifizierungselemente, insbesondere die Querschnittsflächen oder Drahtdurchmesser von Wicklungen, Spulen oder Drähten; vorzugsweise beträgt die Querschnittsfläche oder der Drahtdurchmesser eines elektrisch leitfähigen Identifizierungselements zwischen 0,05 mm bis 1 mm; vorzugsweise unterscheiden sich die Querschnittsflächen oder Drahtdurchmesser, insbesondere eines Sets von Werkzeugen, (jeweils) um 0,05 mm, 0,1 mm, 0,15 mm, 0,2 mm, 0,25 mm oder um 0,5 mm;

- die Dicken der elektrisch leitfähigen Identifizierungselemente quer oder radial zu einer Längsachse des Werkzeugschafts; vorzugsweise beträgt die Dicke eines elektrisch leitfähigen Identifizierungselements zwischen 0,03 mm bis 1 mm, vorzugsweise 0,05 mm bis 0,5 mm; vorzugsweise unterscheiden sich die Dicken, insbesondere eines Sets von Werkzeugen, (jeweils) um 0,05 mm, 0,1 mm, 0,15 mm, 0,2 mm, 0,25 mm oder um 0,5 mm;
- die Schichtstärken der auf den Werkzeugschäften abgeschiedenen elektrisch leitfähigen Identifizierungselemente; vorzugsweise beträgt die Schichtstärke eines elektrisch leitfähigen Identifizierungselements zwischen 0,03 mm bis 1 mm, vorzugsweise 0,05 mm bis 0,5 mm; vorzugsweise unterscheiden sich die Schichtstärken, insbesondere eines Sets von Werkzeugen, (jeweils) um 0,05 mm, 0,1 mm, 0,15 mm, 0,2 mm, 0,25 mm oder um 0,5 mm;
- die Materialien der elektrisch leitfähigen Identifizierungselemente; vorzugsweise weist zumindest ein elektrisch leitfähiges Identifizierungselement eines Werkzeugs ein Material auf, das sich von einem Material eines elektrisch leitfähigen Identifizierungselements eines anderen Werkzeugs unterscheidet; zum Beispiel weist ein elektrisch leitfähiges Identifizierungselement Kupfer, Gold, Silber, Zinn, Wismut oder eine metallische Legierung, vorzugsweise mit zumindest einem der vorgenannten Metalle, zum Beispiel Messing, Bronze oder Neusilber, auf;
- die Windungszahlen und / oder Längen der als Spulen oder Wicklungen ausgebildeten elektrisch leitfähigen Identifizierungselemente; vorzugsweise beträgt die Windungszahl eines elektrisch leitfähigen Identifizierungselements zwischen einer und 50 Windungen; vorzugsweise unterscheiden sich die Windungszahlen, insbesondere eines Sets von Werkzeugen, (jeweils) um 1, 2, 3, 4, 5, 6, 7, 8, 9 oder 10 Windungen.

Vorzugsweise ist / sind (zusätzlich zu dem elektrisch leitfähigen Identifizierungselement) in dem Grundmaterial des Werkzeugs, insbesondere des Werkzeugschafts, durch die elektromagnetische, insbesondere induktive, Kopplung zwischen dem in der Werkzeughalterung aufgenommenen Werkzeug und der mit der periodischen, insbesondere sinusförmigen, Energie versorgten zumindest einen Messspule ein elektrischer Wechselstrom und / oder elektrische Wirbelströme induzierbar, welche ebenfalls zur Generierung des dem jeweiligen Werkzeug zuordenbaren periodischen, insbesondere sinusförmigen, Messsignals, insbesondere durch Veränderung der in der zumindest einen Messspule fließenden periodischen, insbesondere sinusförmigen, elektrischen Energie durch Gegeninduktivität und / oder Ohmsche Verluste, beitragen.

Vorzugsweise unterscheiden sich zur Erkennung oder Unterscheidung der mehreren unterschiedlichen Werkzeuge durch die Auswertevorrichtung die mehreren Werkzeuge, insbesondere des Werkzeugsets, durch zumindest einen der folgenden Parameter:
- das Grundmaterial des Werkzeugschafts; beispielsweise umfasst zumindest ein Werkzeug ein Grundmaterial aus Metall oder aus Kunststoff; beispielsweise umfasst zumindest ein Werkzeug ein Grundmaterial aus einem ersten Metall oder aus einer ersten Metalllegierung und ein anderes Werkzeug ein Grundmaterial aus einem zweiten, anderen Metall oder aus einer zweiten, anderen Metalllegierung;
- den Durchmesser zumindest eines Abschnitts des Werkzeugschafts; vorzugsweise unterscheiden sich die Durchmesser von zumindest Abschnitten mehrerer unterschiedlicher Werkzeugschäfte, insbesondere eines Sets von Werkzeugen, (jeweils) um 0,05 mm, 0,1 mm, 0,15 mm, 0,2 mm;
- die Länge des Werkzeugschafts; vorzugsweise unterscheiden sich die Längen mehrerer unterschiedlicher Werkzeugschäfte, insbesondere eines Sets von Werkzeugen, (jeweils) um 0,05 mm, 0,1 mm, 0,15 mm, 0,2 mm.

Vorzugsweise weist der Werkzeugschaft des zumindest einen Werkzeugs oder zumindest eines Werkzeugs eines Sets von Werkzeugen mehrere, zumindest zwei oder drei oder vier elektrisch leitfähige und voneinander beabstandete Identifizierungselemente auf. Damit ist eine noch zuverlässigere Identifizierung eines Werkzeugs und / oder die Erkennung und Unterscheidung einer noch größeren Anzahl von Werkzeugen möglich. Die voneinander beabstandeten Identifizierungselemente umfassen zum Beispiel einen Draht, eine Wicklung, Spule, Beschichtung, Folie oder Hülse.

Die mehreren voneinander beabstandeten Identifizierungselemente eines Werkzeugschafts können gleiche Elemente (zum Beispiel zwei oder mehrere Drähte, Wicklungen, Spulen, Beschichtungen, Folien oder Hülsen) oder unterschiedliche Elemente (zum Beispiel eine Hülse und eine Wicklung oder eine Folie und eine Spule) aufweisen. Jedes der mehreren voneinander beabstandeten Identifizierungselemente eines Werkzeugschafts kann einen den Werkzeugschaft umgebenden geschlossenen elektrischen Stromkreis bilden, in dem der elektrische Wechselstrom, insbesondere um den Werkzeugschaft oder um eine Längsachse des Werkzeugschafts, fließen kann, oder zwei elektrisch voneinander getrennte Enden aufweisen. Alternativ unterscheiden sich die mehreren voneinander beabstandeten Identifizierungselemente eines Werkzeugschafts dadurch, dass zumindest eines der Identifizierungselemente einen geschlossenen elektrischen Stromkreis bildet und zumindest ein anderes der Identifizierungselemente zwei elektrisch voneinander getrennte Enden aufweist.

Vorzugsweise umfasst die medizinische oder dentale Behandlungsvorrichtung mehrere Messspulen (oder eine Messspule mit zumindest drei Anschlüssen), die mit der Auswertevorrichtung zur Erkennung, ob ein Werkzeug mit der Behandlungsvorrichtung verbunden ist oder welches von mehreren Werkzeugen mit der Behandlungsvorrichtung verbunden ist, elektrisch verbunden sind, so dass die Auswertevorrichtung insbesondere mehrere periodische, insbesondere sinusförmige, Messsignale von den mehreren Messspulen empfangen kann, um die mehreren Werkzeug unterscheiden und erkennen zu können. Dies ist insbesondere dann von Vorteil, wenn der Werkzeugschaft zumindest eines Werkzeugs, insbesondere eines Werkzeugsets, mehrere, zumindest zwei elektrisch leitfähige und voneinander beabstandete Identifizierungselemente aufweist. Vorzugsweise ist jeder Messspule eines der mehreren voneinander beabstandeten Identifizierungselemente zugeordnet, insbesondere wenn das Werkzeug in der Werkzeughalterung aufgenommen ist.

Vorzugsweise ist die medizinische oder dentale Behandlungsvorrichtung ausgebildet, die mehreren Messspulen zeitlich sequentiell mit periodischer, insbesondere sinusförmiger, elektrischer Energie (Wechselstrom) zu versorgen, so dass insbesondere mehrere zeitlich versetzte periodische, insbesondere sinusförmige, Messsignale erzeugbar sind und / oder von der Auswertevorrichtung zur Erkennung eines Werkzeugs ausgewertet werden. Alternativ ist die medizinische oder dentale Behandlungsvorrichtung ausgebildet, die mehreren Messspulen gleichzeitig mit periodischer, insbesondere sinusförmiger, elektrischer Energie (Wechselstrom) zu versorgen, um mehrere periodische, insbesondere sinusförmige, Messsignale zu erzeugen, die von der Auswertevorrichtung zur Erkennung eines Werkzeugs ausgewertet werden.

Alternativ oder zusätzlich ist es auch möglich, dass die medizinische oder dentale Behandlungsvorrichtung oder die Auswertevorrichtung ausgebildet ist, zumindest zwei der mehreren Messspulen zur elektromagnetischen, insbesondere induktiven, Kopplung mit dem in der Werkzeughalterung aufgenommenen Werkzeug, insbesondere dem zumindest einen elektrisch leitfähigen Identifizierungselement, zusammenzuschalten. Dies ist zum Beispiel denkbar, wenn ein Set von Werkzeugen zumindest ein Werkzeug mit einem elektrisch leitfähigen Identifizierungselement und zumindest ein Werkzeug mit mehreren elektrisch leitfähigen Identifizierungselementen aufweist: zur elektromagnetischen, insbesondere induktiven, Kopplung mit dem Werkzeug mit einem elektrisch leitfähigen Identifizierungselement werden die mehreren Messspulen zusammengeschalten und zur elektromagnetischen, insbesondere induktiven, Kopplung mit dem Werkzeug mit mehreren elektrisch leitfähigen Identifizierungselementen werden die mehreren Messspulen nicht zusammengeschalten bzw. wie im Vorstehenden beschrieben, ist jeder Messspule eines der mehreren voneinander beabstandeten Identifizierungselemente zugeordnet.

Selbstverständlich ist es auch möglich, mit nur einer Messspule eine elektromagnetische, insbesondere induktive, Kopplung mit einem Werkzeug mit mehreren elektrisch leitfähigen Identifizierungselement aufzubauen und das Werkzeug korrekt zu erkennen bzw. mit mehreren Messspulen eine elektromagnetische, insbesondere induktive, Kopplung mit einem Werkzeug mit nur einem elektrisch leitfähigen Identifizierungselement aufzubauen und das Werkzeug korrekt zu erkennen.

Vorzugsweise ist die medizinische oder dentale Behandlungsvorrichtung, insbesondere die elektrische Energieversorgungsvorrichtung, ausgebildet, zur Erkennung, ob ein Werkzeug mit der Behandlungsvorrichtung verbunden ist oder welches von mehreren Werkzeugen mit der Behandlungsvorrichtung verbunden ist, die zumindest eine Messspule mit periodischer, insbesondere sinusförmiger, elektrischer Energie (Wechselstrom) mit unterschiedlichen Frequenzen zu versorgen, wodurch die Erkennung oder Unterscheidung der Werkzeuge noch zuverlässiger wird. Vorzugsweise ist die elektrische Energieversorgungsvorrichtung Teil eines Mikrokontrollers oder einer Mikrokontrollereinheit der Behandlungsvorrichtung, wobei die Versorgung der zumindest einen Messspule mit periodischer, insbesondere sinusförmiger, elektrischer Energie mit unterschiedlichen Frequenzen (über Software) durch den Mikrokontroller gesteuert ist. Alternativ ist es auch denkbar, dass die medizinische oder dentale Behandlungsvorrichtung einen mit der elektrischen Energieversorgungsvorrichtung und der zumindest einen Messspule elektrisch verbundenen Frequenzmodulator umfasst, der die Frequenz der an die zumindest eine Messspule abgegebenen periodischen elektrischen Energie verändert.

Vorzugsweise sind die Behandlungsvorrichtung oder der Frequenzmodulator ausgebildet, die zumindest eine Messspule mit periodischer, insbesondere sinusförmiger, elektrischer Energie mit derart unterschiedlichen Frequenzen oder Frequenzbereichen zu versorgen, dass unterschiedliche physikalische Effekte die Generierung des periodischen, insbesondere sinusförmigen, Messsignals (unterschiedlich dominant) beeinflussen, wodurch die Erkennung oder Unterscheidung eines Werkzeugs noch zuverlässiger wird: bevorzugt ist eine Frequenz derart gewählt, zum Beispiel aus einem Frequenzbereich kleiner 500 Hz, dass die Generierung des periodischen, insbesondere sinusförmigen, Messsignals vorwiegend durch Induktivität oder weichmagnetische Eigenschaft des Werkzeugs (des Grundmaterials und / oder des elektrisch leitfähigen Identifizierungselements) bewirkt ist; bevorzugt ist eine Frequenz derart gewählt, zum Beispiel aus einem Frequenzbereichen zwischen 1 kHz und 200 kHz, dass die Generierung des periodischen, insbesondere sinusförmigen, Messsignals vorwiegend durch die Gegeninduktivität des Werkzeugs (insbesondere des elektrisch leitfähigen Identifizierungselements) bewirkt ist; bevorzugt ist eine Frequenz derart gewählt, zum Beispiel aus einem Frequenzbereichen größer 200 kHz, dass die Generierung des periodischen, insbesondere sinusförmigen, Messsignals vorwiegend durch die im Werkzeug induzierten Wirbelströme (insbesondere in dem elektrisch leitfähigen Identifizierungselement) bewirkt ist. Vorzugsweise sind zumindest zwei dieser drei genannten Frequenzen oder Frequenzbereiche in der Behandlungsvorrichtung, zum Beispiel der Auswertevorrichtung oder einer Steuervorrichtung, vorbestimmt oder gespeichert, so dass die zumindest eine Messspule, vorzugsweise automatisiert, mit periodischer, insbesondere sinusförmiger, elektrischer Energie mit entsprechenden Frequenzen versorgbar ist.

Alternativ ist die Behandlungsvorrichtung oder der Frequenzmodulator ausgebildet, die zumindest eine Messspule mit periodischer, insbesondere sinusförmiger, elektrischer Energie mit unterschiedlichen Frequenzen aus zumindest einem der im Vorstehenden genannten Frequenzbereichen zu versorgen. Besonders bevorzugt sind die Behandlungsvorrichtung oder der Frequenzmodulator ausgebildet, die zumindest eine Messspule mit periodischer, insbesondere sinusförmiger, elektrischer Energie (Wechselstrom) mit zumindest zwei unterschiedlichen Frequenzen im Bereich von etwa 1 kHz bis etwa 60 kHz zu versorgen. Damit ist in vorteilhafterweise eine zuverlässige Erkennung und Unterscheidung von Werkzeugen unter Vermeidung von störenden Effekten, zum Beispiel einer zu starken Erhitzung der Behandlungsvorrichtung, möglich.

Alternativ ist die Behandlungsvorrichtung oder der Frequenzmodulator ausgebildet, die zumindest eine Messspule mit periodischer, insbesondere sinusförmiger, elektrischer Energie mit nur einer Frequenzen zu versorgen, zum Beispiel in einem Bereich von etwa 1 kHz bis etwa 150 kHz, vorzugsweise etwa 1 kHz bis etwa 60 kHz. Die Generierung des periodischen, insbesondere sinusförmigen, Messsignals ist hier vorwiegend durch die Gegeninduktivität des Werkzeugs (insbesondere des elektrisch leitfähigen Identifizierungselements) und in geringem Maße durch Wirbelströme bewirkt.

Vorzugsweise ist die Behandlungsvorrichtung oder die Auswertevorrichtung ausgebildet, das dem jeweiligen Werkzeug zuordenbaren periodische, insbesondere sinusförmige, Messsignal mittels Fourier-Transformation auszuwerten, so dass das periodische, insbesondere sinusförmige, Messsignal insbesondere einem bestimmten Werkzeug zuordenbar ist.

Erfindungsgemäß ist ein Verfahren zur Erkennung ob ein Werkzeug mit einer Behandlungsvorrichtung verbunden ist oder welches von mehreren Werkzeugen mit der Behandlungsvorrichtung verbunden ist, vorgesehen, bei dem die zumindest eine Messspule mit periodischer, insbesondere sinusförmiger, elektrischer Energie (Wechselstrom) versorgt wird, aufgrund einer elektromagnetischen, insbesondere induktiven, Kopplung zwischen dem in der Werkzeughalterung aufgenommenen Werkzeug und der mit der periodischen, insbesondere sinusförmigen, Energie versorgten zumindest einen Messspule ein für jedes Werkzeug spezifisches, durch die Auswertevorrichtung dem jeweiligen Werkzeug zuordenbares periodisches, insbesondere sinusförmiges, Messsignal (in der Messspule) generiert und von der Auswertevorrichtung (über die elektrische Verbindung mit der Messspule) empfangen wird, und die Auswertevorrichtung zur Erkennung, ob ein Werkzeug mit der Behandlungsvorrichtung verbunden ist oder welches von mehreren Werkzeugen mit der Behandlungsvorrichtung verbunden ist, die Phasenverschiebung der elektrischen Spannung und des elektrischen Stroms des periodischen, insbesondere sinusförmigen, Messsignals bestimmt. Vorzugsweise ermittelt die Auswertevorrichtung zur Erkennung, ob ein Werkzeug mit der Behandlungsvorrichtung verbunden ist oder welches von mehreren Werkzeugen mit der Behandlungsvorrichtung verbunden ist, auch die Amplitude, insbesondere die Amplitudenextremwerte, der elektrischen Spannung des periodischen, insbesondere sinusförmigen, Messsignals.

Gemäß einem anderen Ausführungsbeispiel ist ein Verfahren zur Erkennung ob ein Werkzeug mit einer Behandlungsvorrichtung verbunden ist oder welches von mehreren Werkzeugen mit der Behandlungsvorrichtung verbunden ist, vorgesehen, bei dem in dem zumindest einen elektrisch leitfähigen Identifizierungselement des zumindest einen Werkzeugs durch die elektromagnetische, insbesondere induktive, Kopplung zwischen diesem in der Werkzeughalterung aufgenommenen Werkzeug und der mit der periodischen, insbesondere sinusförmigen, Energie versorgten zumindest einen Messspule ein elektrischer Wechselstrom und / oder elektrische Wirbelströme induziert wird / werden, der / die zur Generierung des dem jeweiligen Werkzeug zuordenbaren periodischen, insbesondere sinusförmigen, Messsignals auf die in der zumindest einen Messspule fließende periodische, insbesondere sinusförmige, elektrische Energie, insbesondere über Gegeninduktivität und / oder Ohmsche Verluste, (rück)wirkt / (rück)wirken.

Vorzugsweise ist vorgesehen, dass zur Erkennung, ob ein Werkzeug mit der Behandlungsvorrichtung verbunden ist oder welches von mehreren Werkzeugen mit der Behandlungsvorrichtung verbunden ist, die zumindest eine Messspule mit periodischer, insbesondere sinusförmiger, elektrischer Energie (Wechselstrom) unterschiedlicher Frequenzen versorgt wird, so wie dies im Vorstehenden schon im Detail beschrieben ist.

Vorzugsweise ist vorgesehen, dass der durch die elektromagnetische, insbesondere induktive, Kopplung zwischen dem in der Werkzeughalterung aufgenommenen Werkzeug und der mit der periodischen, insbesondere sinusförmigen, Energie versorgten zumindest einen Messspule induzierte (regelmäßig, periodisch seine Polung ändernder) elektrische Wechselstrom, insbesondere in dem zumindest einen elektrisch leitfähigen Identifizierungselement, um den Werkzeugschaft oder um eine Längsachse des Werkzeugschafts fließt. Vorzugsweise ist das zumindest eine elektrisch leitfähige Identifizierungselement dazu als geschlossener elektrischer Stromkreis ausgebildet, so wie dies im Vorstehenden schon im Detail beschrieben ist.

Vorzugsweise ist vorgesehen, dass der Werkzeugschaft des zumindest einen Werkzeugs zumindest zwei elektrisch leitfähige und voneinander beabstandete Identifizierungselemente aufweist, in denen, vorzugsweise sequentiell, zur Erkennung, ob ein Werkzeug mit der Behandlungsvorrichtung verbunden ist oder welches von mehreren Werkzeugen mit der Behandlungsvorrichtung verbunden ist, elektrischer Wechselstrom und / oder elektrische Wirbelströme induziert wird / werden, so wie dies im Vorstehenden schon im Detail beschrieben ist.

Vorzugsweise weist die Behandlungsvorrichtung mehrere Messspulen auf, die elektrisch mit der Auswertevorrichtung verbunden sind und die zur Erkennung, ob ein Werkzeug mit der Behandlungsvorrichtung verbunden ist oder welches von mehreren Werkzeugen mit der Behandlungsvorrichtung verbunden ist, vorzugsweise sequentiell, mit periodischer, insbesondere sinusförmiger, elektrischer Energie (Wechselstrom) versorgt werden, so wie dies im Vorstehenden schon im Detail beschrieben ist.

Vorzugsweise ist vorgesehen, dass die Behandlungsvorrichtung das mit der Werkzeughalterung verbundenen und durch die Auswertevorrichtung erkannte medizinische oder dentale Werkzeug mit darauf abgestimmten Betriebsparametern betreibt und / oder mit darauf abgestimmten Betriebsmitteln und / oder Betriebsmittelmengen versorgt.

Die Erkennung und Unterscheidung der unterschiedlichen Werkzeuge ist bevorzugt dazu vorgesehen, der Behandlungsvorrichtung, insbesondere dem Handgriffelement, der Antriebseinheit oder dem Werkzeug, auf das Werkzeug abgestimmte Betriebsmittel oder Betriebsmittelmengen zukommen zu lassen oder diese mit darauf abgestimmten Betriebsparametern zu betrieben. So weisen zum Beispiel schwingend betriebene Werkzeuge unterschiedliche Resonanzfrequenzen auf, wobei die Schwingungen erzeugende Antriebseinheit abhängig von dem an die Behandlungsvorrichtung angeschlossenen Werkzeug mit unterschiedlichen Versorgungsspannungen versorgt wird, damit das angeschlossene Werkzeug möglichst exakt mit seiner Resonanzfrequenz betrieben werden kann. Vorzugsweise ist die Behandlungsvorrichtung als Zahnsteinentfernungs- oder Scalerbehandlungsvorrichtung ausgebildet, deren Antriebseinheit einen, insbesondere piezoelektrischen, Schwingungserreger umfasst, der in Abhängigkeit des mit der Behandlungsvorrichtung verbundenen und von der Auswertevorrichtung erkannten Werkzeugs mit auf das Werkzeug abgestimmter Antriebsenergie, Antriebsleistung oder Antriebsspannung versorgbar ist.

Vorzugsweise umfasst die Behandlungsvorrichtung, zum Beispiel die Auswertevorrichtung oder eine Steuervorrichtung oder Versorgungseinheit der Behandlungsvorrichtung, zumindest ein Stellelement, zum Beispiel ein Ventil, und / oder zumindest ein Steuer- oder Regelelement, welches das erkannte medizinische oder dentale Werkzeug mit darauf abgestimmten Betriebsparametern betreibt und / oder mit darauf abgestimmten Betriebsmitteln und / oder Betriebsmittelmengen versorgt.

Vorzugsweise benötigen unterschiedliche Werkzeuge unterschiedliche Kühlmittel oder Kühlmittelmengen, so dass die Behandlungsvorrichtung ausgebildet ist, auf das Werkzeug abgestimmte Kühlmittel oder Kühlmittelmengen abzugeben.

Damit das Werkzeug mit den abgestimmten Betriebsparametern, Betriebsmitteln oder Betriebsmittelmengen versorgbar ist, ist die Auswertevorrichtung nach der Erkennung des an die Behandlungsvorrichtung angeschlossenen Werkzeugs vorzugsweise zur Abgabe eines spezifischen Steuersignals für jedes Werkzeug an eine Steuervorrichtung oder Versorgungseinheit ausgebildet. Dazu sind in der Auswertevorrichtung vorzugsweise ein Speicher, in dem für jedes Werkzeug ein charakteristischer Wert hinterlegt ist, insbesondere ein charakteristischer Wert der Phasenverschiebung, insbesondere zusätzlich auch ein Amplitudenwert der Spannung des periodischen, insbesondere sinusförmigen, Messsignals, oder ein davon abgeleiteter Wert, und einen Komparator vorgesehen, der zur Erkennung des mit der Behandlungsvorrichtung verbundenen Werkzeugs den hinterlegten Wert mit dem für jedes Werkzeug spezifischen Wert der Phasenverschiebung, insbesondere zusätzlich auch einem Amplitudenwert der Spannung des periodischen, insbesondere sinusförmigen, Messsignals, oder einem davon abgeleiteten Signal vergleicht. Des Weiteren sind in der Behandlungsvorrichtung für jedes Werkzeug Daten über die benötigten Betriebsmittel oder Betriebsmittelmengen hinterlegt. Auf Basis dieser Daten und des von der Auswertevorrichtung abgegebenen Steuersignals, welches das erkannte Werkzeug definiert, steuert die Steuervorrichtung und / oder die Versorgungseinheit die Betriebsparameter und / oder Abgabe der Betriebsmittel oder Betriebsmittelmengen.

Gemäß einem Ausführungsbeispiel umfasst ein medizinisches oder dentales Werkzeug einen Werkzeugschaft und ein mit dem Werkzeugschaft verbundenes Arbeitsende zum Bearbeiten einer Behandlungsstelle, wobei der Werkzeugschaft zumindest ein elektrisch leitfähiges Identifizierungselement aufweist, in dem durch eine elektromagnetische, insbesondere induktive, Kopplung zwischen dem Werkzeug und einer mit periodischer, insbesondere sinusförmiger, elektrischer Energie (Wechselstrom) versorgten Messspule ein elektrischer Wechselstrom und / oder elektrische Wirbelströme induzierbar ist / sind, der / die vorzugsweise zur Generierung eines dem Werkzeug zuordenbaren periodischen, insbesondere sinusförmigen, Messsignals auf die in der zumindest einen Messspule fließende periodische, insbesondere sinusförmige, elektrische Energie, insbesondere über Gegeninduktivität und / oder Ohmsche Verluste, (rück)wirkt / (rück)wirken.

Bezüglich bevorzugter Merkmale des medizinischen oder dentalen Werkzeugs, insbesondere des zumindest einen elektrisch leitfähigen Identifizierungselements, des Grundmaterials usw., wird zwecks Vermeidung von Wiederholungen auf das Vorstehende verwiesen. Jedes der dort genannten und beschriebenen Merkmale des medizinischen oder dentalen Werkzeugs ist einzeln oder in Kombination auf das hier genannte medizinische oder dentale Werkzeug anwendbar oder übertragbar.

Vorzugsweise ist ein Set von mehreren, insbesondere im Vorstehenden beschriebenen, medizinischen oder dentalen Werkzeugen vorgesehen, wobei sich die elektrisch leitfähigen Identifizierungselemente der mehreren Werkzeuge durch zumindest einen der folgenden Parameter unterscheiden: die axialen Längen der elektrisch leitfähigen Identifizierungselemente in Bezug auf eine Längsachse des Werkzeugschafts; die Querschnittsflächen der elektrisch leitfähigen Identifizierungselemente; die Dicken der elektrisch leitfähigen Identifizierungselemente quer oder radial zu einer Längsachse des Werkzeugschafts; die Schichtstärken der auf den Werkzeugschäften abgeschiedenen elektrisch leitfähigen Identifizierungselemente; die Materialien der elektrisch leitfähigen Identifizierungselemente; die Windungszahlen und / oder Längen der als Spulen oder Wicklungen ausgebildeten elektrisch leitfähigen Identifizierungselemente; die Drahtdurchmesser der als Spulen oder Wicklungen ausgebildeten elektrisch leitfähigen Identifizierungselemente.

Vorzugsweise unterscheiden sich die mehreren Werkzeuge, insbesondere die Werkzeugschäfte, durch zumindest einen der folgenden Parameter: das Grundmaterial des Werkzeugschafts; den Durchmesser des Werkzeugschafts; die Länge des Werkzeugschafts.

Bezüglich bevorzugter Merkmale der medizinischen oder dentalen Werkzeuge des Werkzeugsets, insbesondere des zumindest einen elektrisch leitfähigen Identifizierungselements, des Grundmaterials usw., wird zwecks Vermeidung von Wiederholungen auf das Vorstehende verwiesen. Jedes der dort genannten und beschriebenen Merkmale des medizinischen oder dentalen Werkzeugs ist einzeln oder in Kombination auf die hier genannte medizinischen oder dentale Werkzeuge des Werkzeugsets anwendbar oder übertragbar.

Die Erfindung wird nachfolgend anhand bevorzugter Ausführungsbeispiele und Bezug nehmend auf die beigefügten Zeichnungen erläutert. Es zeigt die
Figur 1 ein Ausführungsbeispiel einer medizinischen oder dentalen Behandlungsvorrichtung mit einer Auswertevorrichtung zur Erkennung ob ein Werkzeug mit der Behandlungsvorrichtung verbunden ist oder welches von mehreren Werkzeugen mit der Behandlungsvorrichtung verbunden ist und einem Handgriffelement zur Zahnsteinentfernung;
Figur 2 eine Schnittdarstellung des werkzeugseitigen Endes des Handgriffelements zur Zahnsteinentfernung der Figur 1;
Figur 3 in schematischer Darstellung ein Ausführungsbeispiel eines Schaltbilds einer medizinischen oder dentalen Behandlungsvorrichtung mit einer Auswertevorrichtung zur Erkennung, ob ein Werkzeug oder welches von mehreren Werkzeugen mit der Behandlungsvorrichtung verbunden ist;
Figur 4 in schematischer Darstellung ein weiteres Ausführungsbeispiel eines Schaltbilds einer medizinischen oder dentalen Behandlungsvorrichtung mit einer Auswertevorrichtung zur Erkennung, ob ein Werkzeug oder welches von mehreren Werkzeugen mit der Behandlungsvorrichtung verbunden ist;
Figur 5 in schematischer Darstellung ein weiteres Ausführungsbeispiel eines Schaltbilds einer medizinischen oder dentalen Behandlungsvorrichtung mit einer Auswertevorrichtung zur Erkennung, ob ein Werkzeug oder welches von mehreren Werkzeugen mit der Behandlungsvorrichtung verbunden ist;
Figur 6 mehrere Werkzeuge oder ein Werkzeugset mit mehreren Werkzeugen mit jeweils einem elektrisch leitfähigen Identifizierungselement in Form von zumindest einer Wicklung;
Figur 7 mehrere Werkzeuge oder ein Werkzeugset mit mehreren Werkzeugen mit jeweils einem elektrisch leitfähigen Identifizierungselement in Form von zumindest einer Spule;
Figur 8 mehrere Werkzeuge oder ein Werkzeugset mit mehreren Werkzeugen mit jeweils einem elektrisch leitfähigen Identifizierungselement in Form von zumindest einer Folie;
Figur 9 mehrere Werkzeuge oder ein Werkzeugset mit mehreren Werkzeugen mit jeweils einem elektrisch leitfähigen Identifizierungselement in Form von zumindest einer Beschichtung;
Figur 10 mehrere Werkzeuge oder ein Werkzeugset mit mehreren Werkzeugen mit jeweils einem elektrisch leitfähigen Identifizierungselement in Form von zumindest einer geschlitzten Hülse;
Figur 11 ein Werkzeug mit zwei voneinander beabstandeten, elektrisch leitfähigen Identifizierungselementen in Form einer Wicklung und einer geschlitzten Hülse;
Figur 12 einen Querschnitt durch die Werkzeugschäfte mehrerer Werkzeuge, insbesondere eines Werkzeugsets, mit jeweils einem elektrisch leitfähigen Identifizierungselement in Form von zumindest einer Beschichtung mit unterschiedlichen Schichtstärken;
Figur 13 zwei schematische Diagramme mit den jeweiligen Phasenverschiebungen und den unterschiedlichen Amplituden zweier unterschiedlicher Werkzeuge zur Unterscheidung der Werkzeuge durch die Auswertevorrichtung.

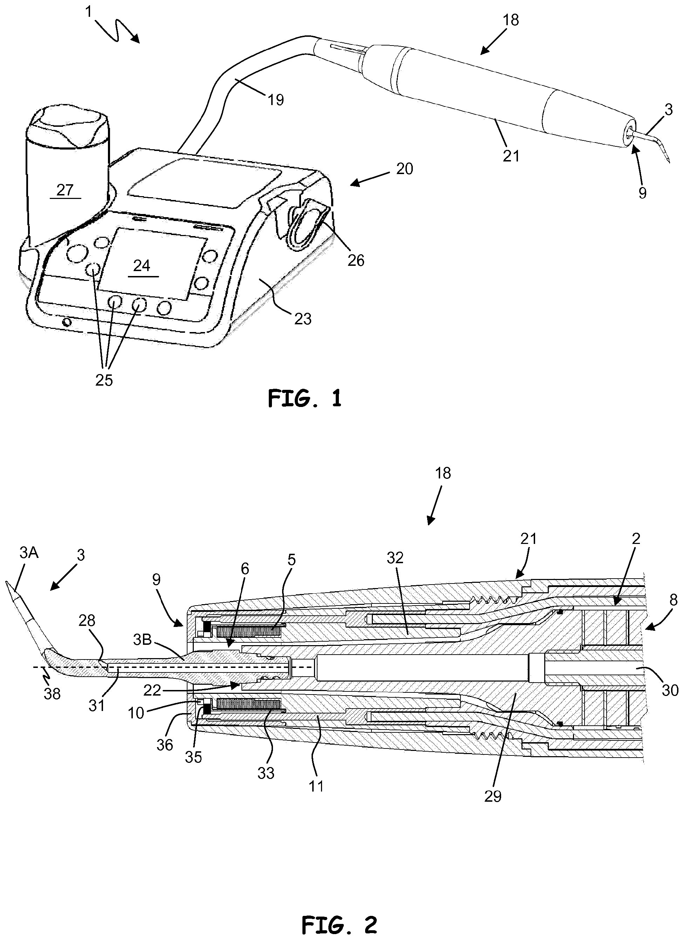

Die in der Figur 1 dargestellte medizinische oder dentale Behandlungsvorrichtung 1 ist als Zahnsteinentfernungs- oder Scalerbehandlungsvorrichtung ausgebildet. Sie umfasst ein Handgriffelement oder Handstück 18, eine Steuervorrichtung oder ein Steuergerät 20 und einen Versorgungs- oder Verbindungsschlauch 19, der das Steuergerät 20 und das Handstück 18 verbindet.

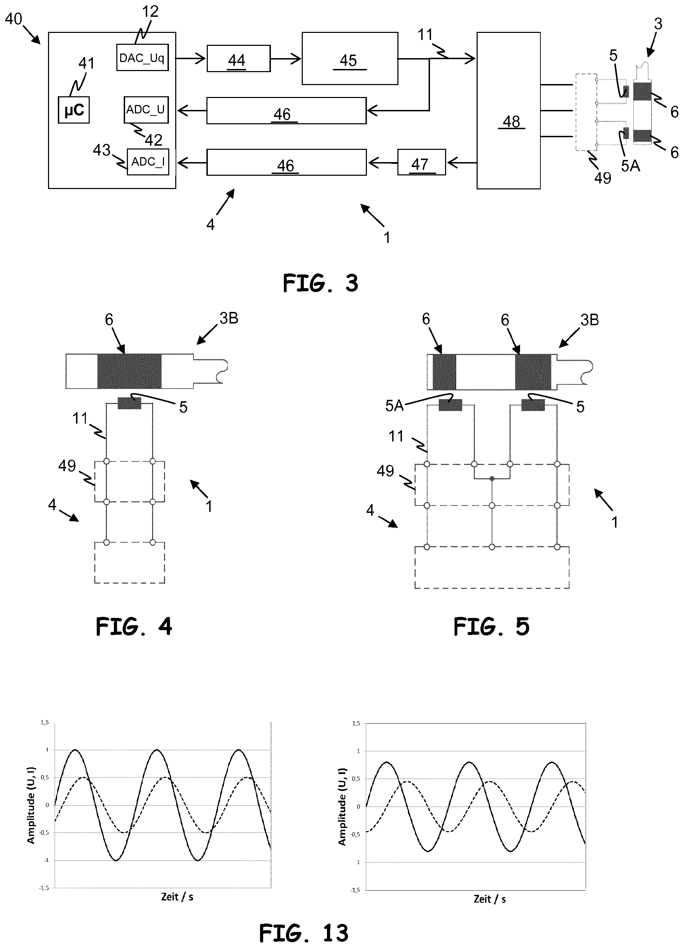

Das in der Figur 2 im Detail dargestellte vorzugsweise gerade, längliche Handstück 18 weist eine zylindrische, hohle Außenhülse 21 auf, in der, wie im Weiteren noch im Detail beschrieben, unter anderem die Antriebseinheit 2 für mit dem Handstück 18 verbindbare Werkzeuge 3, zumindest Teile der Auswertevorrichtung 4 zur Erkennung ob ein Werkzeug mit der Behandlungsvorrichtung verbunden ist oder welches von mehreren Werkzeugen mit der Behandlungsvorrichtung verbunden ist (siehe Figur 3, im Folgenden auch als Werkzeugerkennungsvorrichtung 4 bezeichnet), eine Werkzeugaufnahme oder Werkzeughalterung 22 zur lösbaren Aufnahme mehrerer unterschiedlicher Werkzeuge 3 und eine, vorzugsweise die Werkzeughalterung 22 ringförmig umgebende, Beleuchtungsvorrichtung 9 angeordnet sind. Die Steuervorrichtung oder das Steuergerät 20 weist ein Gehäuse 23 mit einer Anzeige 24 für feststehende oder einstellbare Betriebsparameter oder für das von der Werkzeugerkennungsvorrichtung 4 detektierte Werkzeug 3, ein oder mehrere Stellelemente 25, zum Beispiel Drucktaster, zur Auswahl oder Änderung von Betriebsparametern, eine Handstückablage 26 und eine Flüssigkeitsquelle 27 mit einer Kühl- oder Spülflüssigkeit auf.

Der Versorgungs- oder Verbindungsschlauch 19 enthält mehrere Medien- oder Betriebsmittelleitungen, insbesondere elektrische Leitungen, welche die Antriebseinheit 2 und die Werkzeugerkennungsvorrichtung 4 mit einer elektrischen Energieversorgungsvorrichtung 12 (siehe Figur 3) verbindet. Eine Medienleitung 30 verbindet die Flüssigkeitsquelle 27 mit der Werkzeughalterung 22 und einem darin aufgenommenen Werkzeug 3, so dass über eine Flüssigkeitsabgabeöffnung 28 des Werkzeugs 3 Flüssigkeit auf die Behandlungsstelle und / oder das Werkzeug 3, insbesondere dessen Arbeitsende 3A abgebbar ist.

Wie aus der Figur 2 zu erkennen ist, umfasst die Antriebseinheit 2 einen Schwingungserreger 8, der vorzugsweise einen piezoelektrischen Schwingungserreger mit mehreren Piezoelementen aufweist. Der Schwingungserreger 8 ist zur Schwingungsübertragung über eine Sonotrode 29, die insbesondere als hohler Schwingschaft ausgebildet ist, mit dem Werkzeug 3 verbunden. An einem Ende der Sonotrode 29 ist die Werkzeughalterung 22 zur lösbaren Aufnahme mehrerer unterschiedlicher Werkzeuge 3 vorgesehen. Die Werkzeughalterung 22 umfasst zum Beispiel ein Innengewinde, das mit einem Außengewinde des Werkzeugs 3 das insbesondere an dessen Werkzeugschaft 3B vorgesehen ist, verbindbar ist. Die Werkzeughalterung 22 kann zusätzlich oder alternativ eine konische Reibfläche aufweisen, die mit einer zweiten konischen Reibfläche des Werkzeugs 3, die insbesondere an dessen Werkzeugschaft 3B vorgesehen ist, einen Reibschluss bildet.

Die mit der Flüssigkeitsquelle 27 verbundene Medienleitung 30 mündet in die hohle Sonotrode 29, von der die Kühlflüssigkeit in einen Kanal 31 des Werkzeugs 3 übertritt, um über die Flüssigkeitsabgabeöffnung 28 auszutreten.

Am werkzeugseitigen Ende des Handstücks 18 sind die Sonotrode 29, die Werkzeughalterung 22 und ein darin aufgenommener Werkzeugschaft 3B eines Werkzeugs von der Beleuchtungsvorrichtung 9 umgeben.

Am werkzeugseitigen Ende des Handstücks 18 ist des Weiteren zumindest eine, vorzugsweise zylindrische, Messspule 5, 5A vorgesehen, die Teil der Werkzeugerkennungsvorrichtung 4 (siehe auch Figuren 3 - 5) ist. Die zumindest eine Messspule 5, 5A umgibt insbesondere zumindest einen Abschnitt der Werkzeughalterung 22 oder ist anschließend an die Werkzeughalterung 22 angeordnet. Die zumindest eine Messspule 5, 5A ist vorzugsweise derart in dem Handstück 18 angeordnet, dass, wenn ein Werkzeug 3 in der Werkzeughalterung 22 aufgenommen ist, ein elektrisch leitfähiges Identifizierungselement 6 des Werkzeugs 3 nahe an der zumindest einen Messspule 5, 5A, insbesondere innerhalb der zumindest eine Messspule 5, 5A, positioniert ist, so dass aufgrund einer elektromagnetischen, insbesondere induktiven, Kopplung zwischen dem in der Werkzeughalterung 22 aufgenommenen Werkzeug 3, insbesondere dessen zumindest einen elektrisch leitfähigen Identifizierungselements 6, und der mit periodischer, insbesondere sinusförmiger, elektrischer Energie (Wechselstrom) der elektrischen Energieversorgungsvorrichtung 12 versorgten zumindest einen Messspule 5, 5A ein für jedes von mehreren Werkzeugen 3 spezifisches, durch die Auswertevorrichtung 4 dem jeweiligen Werkzeug 3 zuordenbares periodisches, insbesondere sinusförmiges, Messsignal generierbar ist.

Die zumindest eine Messspule 5, 5A ist zum Beispiel auf einer Trägerhülse 32 aus Kunststoff gelagert. Vorzugsweise umgibt ein magnetisches Rückschlusselement 33, das insbesondere aus Metallblech besteht, die zumindest eine Messspule 5, 5A, insbesondere an ihrer Außenseite und bevorzugt zumindest teilweise auch an ihrer dem Werkzeugschaft 3B und der Sonotrode 29 zugewandten Innenseite. Dieses hülsenartige, mit einer Öffnung nach Innen versehene Rückschlusselement 33 bewirkt eine Bündelung oder Verdichtung der Magnetfeldlinien oder eine Erhöhung der magnetischen Flussdichte, insbesondere in dem zumindest einen elektrisch leitfähigen Identifizierungselements 6 des Werkzeugschafts 3B des in der Werkzeughalterung 22 aufgenommenen Werkzeugs 3, wodurch die Erkennung des Werkzeugs 3 durch die Werkzeugerkennungsvorrichtung 4 begünstigt wird. Das magnetische Rückschlusselement 33 unterstützt insbesondere auch die Detektierbarkeit mehrerer elektrisch leitfähiger Identifizierungselemente 6.

Vorzugsweise ist die zumindest eine Messspule 5, 5A, insbesondere auch das Rückschlusselement 33, mit einem Vergussmaterial, zum Beispiel mit einem Kunstharz, insbesondere mit Epoxidharz, vergossen, um sie vor äußeren Einflüssen und Verschmutzungen zu schützen und ihre Formstabilität zu gewährleisten.

Distal anschließend an die zumindest eine Messspule 5, 5A ist die Beleuchtungsvorrichtung 9 angeordnet, die eine Platine 35 und eine oder mehrere optische Halbleiterelemente 10, insbesondere Leuchtdioden, umfasst. Die Platine 35 ist ringförmig geformt, so dass durch ihre zentrale Öffnung ein in der Werkzeughalterung 22 aufgenommenes Werkzeug 3 ragen kann. Eine transparente Kunststoffhülse 36 deckt die Platine 35 und die Leuchtdioden 10 ab und schützt sie vor Verschmutzungen und mechanischen Belastungen.

Die elektrische Versorgung der zumindest eine Messspule 5, 5A mit elektrischem Strom der elektrischen Energieversorgungsvorrichtung 12, vorzugsweise auch der Beleuchtungsvorrichtung 9, erfolgt in dem Handstück 18 über eine elektrische Leitung 11. Die elektrische Leitung 11 ist über eine elektrische Leitung in dem Versorgungs- oder Verbindungsschlauch 19 mit der der elektrischen Energieversorgungsvorrichtung 12 verbunden.

Die in der Figur 4 in Form eines schematischen Schaltbilds dargestellte medizinische oder dentale Behandlungsvorrichtung 1 umfasst eine Auswertevorrichtung 4 zur Erkennung, ob ein Werkzeug oder welches von mehreren Werkzeugen mit der Behandlungsvorrichtung verbunden ist, mit einer (einzigen) Messspule 5 und ein (einziges) elektrisch leitfähiges Identifizierungselement 6 am Werkzeugschaft 3B des Werkzeugs 3. Über eine elektrische Leitung 11 ist die Messspule 5 mit der elektrischen Energieversorgungsvorrichtung 12 und den weiteren Komponenten der Auswertevorrichtung 4 verbunden.

Die in der Figur 5 in Form eines schematischen Schaltbilds dargestellte medizinische oder dentale Behandlungsvorrichtung 1 umfasst eine Auswertevorrichtung 4 zur Erkennung, ob ein Werkzeug oder welches von mehreren Werkzeugen mit der Behandlungsvorrichtung verbunden ist, mit zwei Messspulen 5, 5A und zwei voneinander beabstandeten, elektrisch leitfähigen Identifizierungselementen 6 am Werkzeugschaft 3B des Werkzeugs 3. Vorzugsweise sind die beiden elektrisch leitfähigen Identifizierungselement 6 jeweils als eigenständige elektrische Stromkreise 37 ausgebildet. Über eine elektrische Leitung 11 sind die Messspulen 5 miteinander, mit der elektrischen Energieversorgungsvorrichtung 12 und den weiteren Komponenten der Auswertevorrichtung 4 verbunden.

Vorzugsweise ist auch die Beleuchtungsvorrichtung 9 über die elektrische Leitung 11 mit der elektrischen Energieversorgungsvorrichtung 12 zur elektrischen Versorgung der optischen Halbleiterelemente 10 verbunden. Bevorzugt ist die elektrische Leitung 11 mit einer elektrischen Energieversorgungsvorrichtung 12 verbindbar oder verbunden, die zur Versorgung der Beleuchtungsvorrichtung 9 Gleichspannung und zur Versorgung der Spule der Werkzeugerkennungsvorrichtung 4 Wechselspannung bereitstellt. Damit die elektrische Leitung 11 die Beleuchtungsvorrichtung 9 mit Gleichspannung und die zumindest eine Messspule 5 mit Wechselspannung versorgen kann, wird die Wechselspannung der Gleichspannung aufmoduliert oder überlagern die beiden Spannungen einander. Alternativ ist es auch möglich, die Beleuchtungsvorrichtung 9 mit Wechselspannung der elektrischen Energieversorgungsvorrichtung 12 über die elektrische Leitung 11 zu versorgen.

Vorzugsweise ist die Beleuchtungsvorrichtung 9, insbesondere zumindest ein optisches Halbleiterelement 10, auf einem Träger 49, zum Beispiel die Platine 35, angeordnet. Besonders bevorzugt ist der Träger 49 auch Teil der Werkzeugerkennungs- oder Auswertevorrichtung 4 und ist zum Beispiel mit den Spulenenden der zumindest einen Messspule 5 verbunden oder trägt zumindest ein Element der Auswertevorrichtung 4.

Die Figur 3 zeigt in Form eines schematischen Schaltbilds den Aufbau der Werkzeugerkennungsvorrichtung 4, insbesondere der Figur 5, detaillierter.

Die Auswertevorrichtung 4 zur Erkennung, ob ein Werkzeug oder welches von mehreren Werkzeugen mit der Behandlungsvorrichtung 1 verbunden ist, umfasst eine Mikrokontroller-Einheit 40, die zum Beispiel in der Steuervorrichtung oder dem Steuergerät 20, angeordnet ist oder zumindest einen Teil der Steuervorrichtung oder des Steuergeräts 20 bildet. Die Mikrokontroller-Einheit 40 umfasst einen Mikrokontroller 41, die elektrische Energieversorgungsvorrichtung 12 zur elektrischen Versorgung der zumindest einen Messspule 5, vorzugsweise auch der Beleuchtungsvorrichtung 9, eine Vorrichtung zur Spannungsmessung 42 und eine Vorrichtung zur Strommessung 43, vorzugsweise indirekt über eine Spannungsmessung. Die genannten Elemente 12, 42, 43 sind vorzugsweise als Teil des Mikrokontrollers 41 ausgebildet und / oder durch den Mikrokontroller 41 mittels Software gesteuert.

Die Vorrichtung zur Spannungsmessung 42 und die Vorrichtung zur Strommessung 43 sind insbesondere dazu vorgesehen, die elektrische Spannung und den elektrischen Strom(wert) des von der zumindest einen Messspule 5 empfangenen periodischen, insbesondere sinusförmigen, Messsignals zu bestimmen, woraus die Phasenverschiebung zur Erkennung, ob ein Werkzeug 3 oder welches von mehreren Werkzeugen 3 mit der Behandlungsvorrichtung 1 verbunden ist, ermittelt wird. Vorzugsweise ist die Vorrichtung zur Spannungsmessung 42 auch dazu vorgesehen zur Werkzeugerkennung die Amplitude, insbesondere die Amplitudenextremwerte, der elektrischen Spannung des periodischen, insbesondere sinusförmigen, Messsignals zu bestimmen.

Zwischen der zumindest einen Messspule 5 und der elektrischen Energieversorgungsvorrichtung 12 sind vorzugsweise weitere elektrische oder elektronische Bauteil vorgesehen, zum Beispiel ein Filter 44 zum Filtern oder Glätten (der durch den Mikrokontroller der elektrischen Energieversorgungsvorrichtung 12 verursachten hochfrequenten transienten Sprünge) der von der elektrischen Energieversorgungsvorrichtung 12 zur Verfügung gestellten periodischen, insbesondere sinusförmigen, elektrischen Energie und / oder ein Verstärker 45 zum Verstärken der periodischen elektrischen Energie (des Wechselstroms).

Eine Vorrichtung zur Pegelanpassung der Spannung und / oder ein Filter 46 schützen die Mikrokontroller-Einheit 40 und deren Komponenten vor zu hohen Spannungswerten des von der zumindest einen Messspule 5 übertragenen periodisches, insbesondere sinusförmiges, Messsignals. Ein Strom-Spannungswandler 47 bestimmt den Spannungswert des periodischen, insbesondere sinusförmigen, Messsignals aus dem gemessenen Stromwert des periodischen, insbesondere sinusförmigen, Messsignals.

Da die Behandlungsvorrichtung 1 der Figur 3 zwei Messspulen 5, 5A, jedoch nur eine Auswertevorrichtung 4 oder Mikrokontroller-Einheit 40 aufweist, ist es notwendig, zur Werkzeugerkennung die beiden Messspulen 5, 5A sequentiell mit periodischer, insbesondere sinusförmiger, elektrischer Energie (Wechselstrom) zu versorgen. Dazu ist eine Schaltvorrichtung 48 vorgesehen, die mit den jeweils zwei Spulenenden der beiden Messspulen 5, 5A elektrisch verbunden ist und die ausgebildet ist, die beiden Messspulen 5, 5A sequentiell mit der periodischen, insbesondere sinusförmigen, elektrischen Energie der elektrischen Energieversorgungsvorrichtung 12 zu versorgen und insbesondere auch mit der Mikrokontroller-Einheit 40, dem Mikrokontroller 41, der Vorrichtung zur Spannungsmessung 42 und / oder der Vorrichtung zur Strommessung 43 zu verbinden. Die Schaltvorrichtung 48 ist vorzugsweise auf dem Träger 49 angeordnet (siehe auch Figur 5).

Die Schaltvorrichtung 48 ist klarerweise ein optionales Element, das zum Beispiel nicht benötigt wird, wenn die Behandlungsvorrichtung 1 nur eine Messspule 5 aufweist (siehe Figur 4) oder wenn die Behandlungsvorrichtung 1 mehrere Messspulen 5, 5A aufweist und für jede dieser Messspulen 5, 5A zumindest eine eigene Vorrichtung zur Spannungsmessung 42 und eine eigene Vorrichtung zur Strommessung 43 oder eine eigene Mikrokontroller-Einheit 40 vorhanden ist.

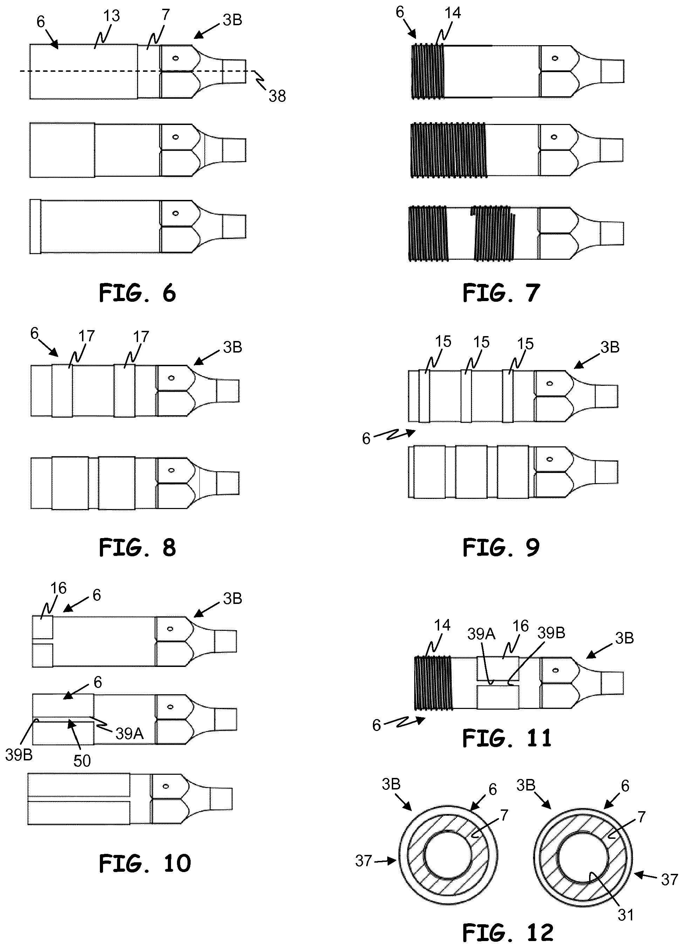

Die Figuren 6 - 12 zeigen unterschiedliche Werkzeuge 3, die insbesondere zur Verwendung mit einer Behandlungsvorrichtung 1 vorgesehen sind. Zwei oder mehr oder alle der dargestellten Werkezeuge können ein Werkzeugset bilden. Jedes der abgebildeten Werkzeuge weist zumindest ein elektrisch leitfähiges Identifizierungselement 6 auf, so dass es durch die Auswertevorrichtung 4 zur Erkennung ob ein Werkzeug mit der Behandlungsvorrichtung 1 verbunden ist oder welches von mehreren Werkzeugen mit der Behandlungsvorrichtung 1 verbunden ist identifizierbar ist. Das elektrisch leitfähige Identifizierungselement 6 ist ein separates Element, das auf einem Grundmaterial 7 jedes Werkzeugs auf dem Werkzeugschaft 3B vorgesehen ist.

Bei den Werkzeugen der Figur 6 ist das elektrisch leitfähige Identifizierungselement 6 jeweils als Wicklung 13 ausgebildet. Die Wicklungen 13 umfassen insbesondere jeweils nur eine einzige Windung. Die Wicklungen 13 sind insbesondere jeweils als geschlossener elektrischer Stromkreis 37 (siehe auch Figur 12) ausgebildet, in dem ein um den Werkzeugschaft 3B und / oder die Längsachse 38 des Werkzeugschaft 3B fließender Wechselstrom induzierbar ist. Die Wicklungen 13 der drei unterschiedlichen Werkzeuge der Figur 6 unterscheiden sich insbesondere in ihren axialen Längen (bezogen auf die Längsachse 38), wodurch sie unterschiedliche Phasenverschiebungen und / oder Amplituden des periodischen, insbesondere sinusförmigen, Messsignals bewirken und damit für die Auswertevorrichtung 4 unterscheidbar sind. Aufgrund der großen axialen Ausdehnung der einzigen Windung der Wicklung 13 bilden zumindest einige der Wicklungen 13 der unterschiedlichen Werkzeuge 3 Hülsen oder hülsenförmige Elemente. Selbstverständlich können sich die Wicklungen 13 auch noch in weiteren, insbesondere im Vorstehenden genannten, Eigenschaften voneinander unterscheiden, zum Beispiel in ihrem Material, in ihrer Dicke und / oder in ihrer radialen Ausdehnung (bezogen auf die Längsachse 38).

Bei den Werkzeugen der Figur 7 ist das elektrisch leitfähige Identifizierungselement 6 jeweils als Spule 14 ausgebildet. Die Spule 14 kann zum Beispiel einen isolierten Wicklungsdraht oder eine flexible, bedruckte Leiterplatte aufweisen. Die Spulen 14 umfassen insbesondere mehrere Windungen. Die Spulen 14 sind insbesondere jeweils als geschlossener elektrischer Stromkreis 37 ausgebildet, in dem ein um den Werkzeugschaft 3B und / oder die Längsachse 38 des Werkzeugschaft 3B fließender Wechselstrom induzierbar ist. Die Spulen 14 der drei unterschiedlichen Werkzeuge der Figur 7 unterscheiden sich insbesondere in ihren axialen Längen (bezogen auf die Längsachse 38) und / oder in der Anzahl der Windungen als auch in der Anzahl der Spulen 14. Während die ersten beiden Werkzeuge 3 jeweils nur eine Spule 14 aufweisen, hat das dritte Werkzeug 3 zwei axial voneinander beabstandetet Spulen 14 zur Erkennung durch die Auswertevorrichtung 4. Selbstverständlich können sich die Spulen 14 wiederum in weiteren, insbesondere im Vorstehenden genannten, Eigenschaften voneinander unterscheiden, zum Beispiel in ihrem Material und / oder in ihren Querschnittsflächen.

Bei den Werkzeugen der Figur 8 ist das elektrisch leitfähige Identifizierungselement 6 jeweils als Folie 17 ausgebildet, wobei jeweils zwei voneinander beabstandete Folien 17 auf einem Werkzeugschaft 3B angeordnet sind. Die Folien 17 sind insbesondere jeweils als geschlossener elektrischer Stromkreis 37 ausgebildet, in dem ein um den Werkzeugschaft 3B und / oder die Längsachse 38 des Werkzeugschaft 3B fließender Wechselstrom induzierbar ist. Die Folien 17 der unterschiedlichen Werkzeuge der Figur 8 unterscheiden sich insbesondere in ihren axialen Längen (bezogen auf die Längsachse 38), wodurch sie unterschiedliche Phasenverschiebungen und / oder Amplituden des periodischen, insbesondere sinusförmigen, Messsignals bewirken und damit für die Auswertevorrichtung 4 unterscheidbar sind. Aufgrund der großen axialen Ausdehnung der Folien 17 bilden zumindest einige dieser Folien 17 der unterschiedlichen Werkzeuge 3 Hülsen oder hülsenförmige Elemente. Selbstverständlich können sich die Folien 17 auch noch in weiteren, insbesondere im Vorstehenden genannten, Eigenschaften voneinander unterscheiden, zum Beispiel in ihrem Material, in ihrer Dicke und / oder in ihrer radialen Ausdehnung (bezogen auf die Längsachse 38).

Bei den Werkzeugen der Figur 9 ist das elektrisch leitfähige Identifizierungselement 6 jeweils als Beschichtung 15 ausgebildet, wobei jeweils drei axial voneinander beabstandete Beschichtungen 15 auf einem Werkzeugschaft 3B angeordnet sind. Die Beschichtungen 15 sind insbesondere jeweils als geschlossener elektrischer Stromkreis 37 ausgebildet, in dem ein um den Werkzeugschaft 3B und / oder die Längsachse 38 des Werkzeugschaft 3B fließender Wechselstrom induzierbar ist. Die Beschichtungen 15 der unterschiedlichen Werkzeuge der Figur 9 unterscheiden sich wiederum insbesondere in ihren axialen Längen (bezogen auf die Längsachse 38), wodurch sie unterschiedliche Phasenverschiebungen und / oder Amplituden des periodischen, insbesondere sinusförmigen, Messsignals bewirken und damit für die Auswertevorrichtung 4 unterscheidbar sind. Selbstverständlich können sich die Beschichtungen 15 auch noch in weiteren, insbesondere im Vorstehenden genannten, Eigenschaften voneinander unterscheiden, zum Beispiel in ihrem Material und / oder in ihrer Schichtdicke.

Bei den Werkzeugen der Figur 10 ist das elektrisch leitfähige Identifizierungselement 6 jeweils als, insbesondere geschlitzte, Hülse 16 ausgebildet. Die geschlitzten Hülsen 16 weisen zwei voneinander elektrisch getrennte Enden 39A, 39B auf, die insbesondere durch den zwischen den beiden Enden 39A, 39B vorgesehenen Abstand oder Schlitz 50 gebildet sind. Die geschlitzten Hülsen 16 bilden somit keinen geschlossenen elektrischen Stromkreis, so das in den geschlitzten Hülsen 16 ausschließlich Wirbelströme induzierbar sind. Die Hülsen 16 der unterschiedlichen Werkzeuge der Figur 10 unterscheiden sich wiederum insbesondere in ihren axialen Längen (bezogen auf die Längsachse 38), wodurch sie unterschiedliche Phasenverschiebungen und / oder Amplituden des periodischen, insbesondere sinusförmigen, Messsignals bewirken und damit für die Auswertevorrichtung 4 unterscheidbar sind. Selbstverständlich können sich die Beschichtungen 15 auch noch in weiteren, insbesondere im Vorstehenden genannten, Eigenschaften voneinander unterscheiden, zum Beispiel in ihrem Material, in ihrer Dicke und / oder in ihrer radialen Ausdehnung (bezogen auf die Längsachse 38).

Das Werkzeug der Figur 11 umfasst zwei unterschiedliche elektrisch leitfähige Identifizierungselemente 6, beispielhaft sind eine Spule 14 und eine geschlitzte Hülse 16 dargestellt. Selbstverständlich können auch andere im Vorstehenden genannte elektrisch leitfähige Identifizierungselemente 6 auf einem Werkzeug 3 miteinander kombiniert werden, wobei jede mögliche Kombination von zwei oder mehr der im Vorstehenden genannten elektrisch leitfähigen Identifizierungselemente 6 denkbar ist.

Aufgrund des Querschnitts durch den Werkzeugschaft 3B der in der Figur 12 dargestellten Werkzeuge 3 ist besonders gut erkennbar, dass die elektrisch leitfähigen Identifizierungselemente 6 sich in ihrer Dicke, Schichtdicke und / oder radialen Ausdehnung (bezogen auf die Längsachse 38) unterscheiden, wodurch sie unterschiedliche Phasenverschiebungen und / oder Amplituden des periodischen, insbesondere sinusförmigen, Messsignals bewirken und damit für die Auswertevorrichtung 4 unterscheidbar sind. Es ist des Weiteren gut zu erkennen, dass die elektrisch leitfähigen Identifizierungselemente 6 als geschlossener elektrischer Stromkreis 37 ausgebildet sind, in dem ein um den Werkzeugschaft 3B und / oder die Längsachse 38 des Werkzeugschaft 3B fließender Wechselstrom induzierbar ist. Selbstverständlich können sich die elektrisch leitfähigen Identifizierungselemente 6 wiederum in weiteren, insbesondere im Vorstehenden genannten, Eigenschaften voneinander unterscheiden, zum Beispiel in ihrem Material und / oder in ihrer axialen Ausdehnung (bezogen auf die Längsachse 38).

Die Figur 13 zweigt zwei schematische Diagramme mit den jeweiligen Phasenverschiebungen und den unterschiedlichen Amplituden zweier unterschiedlicher Werkzeuge 3 zur Unterscheidung der Werkzeuge durch die Auswertevorrichtung 4. Wie im Vorstehenden ausführlich beschrieben, werden die Phasenverschiebungen und die unterschiedlichen Amplituden insbesondere durch das für jedes Werkzeug 3 spezifische elektrisch leitfähige Identifizierungselement 6, vorzugsweise zusätzlich durch das Grundmaterial des Werkzeugs 3, bewirkt. In beiden Diagrammen ist die Phasenverschiebung deutlich zu erkennen: Das als gestrichelte Kurve dargestellte Stromsignal folgt dem als durchgehende Kurve dargestellten Spannungssignal zeitlich versetzt. Die periodischen, insbesondere sinusförmigen, Messsignale der beiden Diagramme, d.h. der beiden durch die Auswertevorrichtung 4 erkannten Werkzeuge, unterscheiden sich in ihren Phasenverschiebungen und in ihren Amplituden, insbesondere den Amplitudenextremwerten oder Amplitudenhöhen.

Die beschriebenen oder dargestellten Ausführungsbeispiele dienen insbesondere der Veranschaulichung der Erfindung. Die in einem Ausführungsbeispiel offenbarten Merkmale sind daher nicht auf dieses Ausführungsbeispiel beschränkt, sondern sind einzeln oder gemeinsam mit einem oder mehreren Merkmalen eines anderen Ausführungsbeispiels kombinierbar.

## Patentansprüche

1. Medizinische oder dentale Behandlungsvorrichtung (1), die mit mehreren unterschiedlichen Werkzeugen (3) verbindbar ist, wobei die medizinische oder dentale Behandlungsvorrichtung (1) umfasst: eine Werkzeughalterung (22) zum Verbinden der Behandlungsvorrichtung (1) mit einem Werkzeug, zumindest eine Messspule (5, 5A), die über eine elektrische Leitung (11) mit einer elektrischen Energieversorgungsvorrichtung (12) verbunden ist und von der elektrischen Energieversorgungsvorrichtung (12) mit einer periodischen, insbesondere sinusförmigen, elektrischen Energie versorgbar ist, und eine mit der zumindest einen Messspule (5, 5A) elektrisch verbundene Auswertevorrichtung (4) zur Erkennung ob ein Werkzeug mit der Behandlungsvorrichtung (1) verbunden ist oder welches von mehreren Werkzeugen mit der Behandlungsvorrichtung (1) verbunden ist, wobei aufgrund einer induktiven Kopplung zwischen dem in der Werkzeughalterung (22) aufgenommenen Werkzeug (3) und der mit der periodischen elektrischen Energie versorgten zumindest einen Messspule (5, 5A) ein für jedes Werkzeug (3) spezifisches, durch die Auswertevorrichtung (4) dem jeweiligen Werkzeug (3) zuordenbares periodisches, insbesondere sinusförmiges, Messsignal generierbar ist, **dadurch gekennzeichnet, dass**
die Auswertevorrichtung (4) zur Erkennung, ob ein Werkzeug mit der Behandlungsvorrichtung (1) verbunden ist oder welches von mehreren Werkzeugen mit der Behandlungsvorrichtung (1) verbunden ist, die Phasenverschiebung der elektrischen Spannung und des elektrischen Stroms des periodischen, insbesondere sinusförmigen, Messsignals bestimmt.

2. Medizinische oder dentale Behandlungsvorrichtung (1) nach Anspruch 1, **dadurch gekennzeichnet, dass**
die Auswertevorrichtung (4) zur Erkennung, ob ein Werkzeug mit der Behandlungsvorrichtung (1) verbunden ist oder welches von mehreren Werkzeugen mit der Behandlungsvorrichtung (1) verbunden ist, die Amplitude, insbesondere die Amplitudenextremwerte, der elektrischen Spannung des periodischen, insbesondere sinusförmigen, Messsignals bestimmt.

3. Medizinische oder dentale Behandlungsvorrichtung (1) nach Anspruch 1 oder 2 mit zumindest einem Werkzeug (3), das mit der Behandlungsvorrichtung (1) verbindbar ist, **dadurch gekennzeichnet, dass**
das zumindest eine Werkzeug (3) einen Werkzeugschaft (3B) und ein mit dem Werkzeugschaft (3B) verbundenes Arbeitsende (3A) zum Bearbeiten einer Behandlungsstelle aufweist, wobei der Werkzeugschaft (3B) zumindest ein elektrisch leitfähiges Identifizierungselement (6) aufweist, in dem durch die induktive Kopplung zwischen dem in der Werkzeughalterung (22) aufgenommenen Werkzeug (3) und der mit der periodischen, insbesondere sinusförmigen, Energie versorgten zumindest einen Messspule (5, 5A) ein elektrischer Wechselstrom und / oder elektrische Wirbelströme induzierbar ist / sind, der / die zur Generierung des dem jeweiligen Werkzeug (3) zuordenbaren periodischen, insbesondere sinusförmigen, Messsignals auf die in der zumindest einen Messspule (5, 5A) fließende periodische, insbesondere sinusförmige, elektrische Energie, insbesondere über Gegeninduktivität und / oder Ohmsche Verluste, rückwirkt / rückwirken.

4. Medizinische oder dentale Behandlungsvorrichtung (1) nach Anspruch 3, **dadurch gekennzeichnet, dass**
das zumindest eine elektrisch leitfähige Identifizierungselement (6) auf einem Grundmaterial (7) des Werkzeugschafts (3B) vorgesehen ist, wobei das zumindest eine elektrisch leitfähige Identifizierungselement (6) und das Grundmaterial (7) unterschiedliche Materialien aufweisen.

5. Medizinische oder dentale Behandlungsvorrichtung (1) nach Anspruch 3 oder 4, **dadurch gekennzeichnet, dass**
das zumindest eine elektrisch leitfähige Identifizierungselement (6) einen Draht, eine Wicklung (13), Spule (14), Beschichtung (15), Folie (17) oder Hülse (16) aufweist.

6. Medizinische oder dentale Behandlungsvorrichtung (1) nach einem der Ansprüche 3, 4 oder 5, **dadurch gekennzeichnet, dass**
das zumindest eine elektrisch leitfähige Identifizierungselement (6) einen den Werkzeugschaft (3B) umgebenden geschlossenen elektrischen Stromkreis (37) bildet, in dem der elektrische Wechselstrom, insbesondere um den Werkzeugschaft (3B) oder um eine Längsachse (38) des Werkzeugschafts (3B), fließen kann.

7. Medizinische oder dentale Behandlungsvorrichtung (1) nach einem der Ansprüche 3, 4 oder 5, **dadurch gekennzeichnet, dass**
das zumindest eine elektrisch leitfähige Identifizierungselement (6) zwei elektrisch voneinander getrennte Enden (39A, 39B) aufweist.

8. Medizinische oder dentale Behandlungsvorrichtung (1) nach einem der Ansprüche 3 - 7, **gekennzeichnet durch**
mehrere unterschiedliche Werkzeuge (3), von denen jedes einen Werkzeugschaft (3B) mit zumindest einem elektrisch leitfähigen Identifizierungselement (6) aufweist, wobei zur Erkennung oder Unterscheidung der Werkzeuge (3) durch die Auswertevorrichtung (4) sich die elektrisch leitfähigen Identifizierungselemente (6) der mehreren Werkzeuge (3) durch zumindest einen der folgenden Parameter unterscheiden: die axialen Längen der elektrisch leitfähigen Identifizierungselemente (6) in Bezug auf eine Längsachse (38) des Werkzeugschafts (3B); die Querschnittsflächen der elektrisch leitfähigen Identifizierungselemente (6); die Dicken der elektrisch leitfähigen Identifizierungselemente (6) quer oder radial zu einer Längsachse (38) des Werkzeugschafts (3B); die Schichtstärken der auf den Werkzeugschäften (3B) abgeschiedenen elektrisch leitfähigen Identifizierungselemente (6); die Materialien der elektrisch leitfähigen Identifizierungselemente (6); die Windungszahlen und / oder Längen der als Spulen (14) oder Wicklungen (13) ausgebildeten elektrisch leitfähigen Identifizierungselemente (6); die Drahtdurchmesser der als Spulen (14) oder Wicklungen (13) ausgebildeten elektrisch leitfähigen Identifizierungselemente (6).

9. Medizinische oder dentale Behandlungsvorrichtung (1) nach Anspruch 8, **dadurch gekennzeichnet, dass**
zur Erkennung oder Unterscheidung der mehreren unterschiedlichen Werkzeuge (3) durch die Auswertevorrichtung (4) sich die mehreren Werkzeuge (3) durch zumindest einen der folgenden Parameter unterscheiden: das Grundmaterial (7) des Werkzeugschafts (3B); den Durchmesser zumindest eines Abschnitts des Werkzeugschafts (3B); die Länge des Werkzeugschafts (3B).

10. Medizinische oder dentale Behandlungsvorrichtung (1) nach einem der Ansprüche 3 - 9, **dadurch gekennzeichnet, dass**
der Werkzeugschaft (3B) des zumindest einen Werkzeugs (3) zumindest zwei elektrisch leitfähige und voneinander beabstandete Identifizierungselemente (6) aufweist.

11. Medizinische oder dentale Behandlungsvorrichtung (1) nach einem der vorstehenden Ansprüche, **gekennzeichnet durch**
mehrere Messspulen (5, 5A), die mit der Auswertevorrichtung (4) zur Erkennung, ob ein Werkzeug mit der Behandlungsvorrichtung (1) verbunden ist oder welches von mehreren Werkzeugen mit der Behandlungsvorrichtung (1) verbunden ist, elektrisch verbunden sind.

12. Medizinische oder dentale Behandlungsvorrichtung (1) nach einem der vorstehenden Ansprüche, **gekennzeichnet durch**
eine elektrische Energieversorgungsvorrichtung (12), die ausgebildet ist, der zumindest einen Messspule (5, 5A) periodische, insbesondere sinusförmige, elektrischer Energie mit unterschiedlichen Frequenzen zur Verfügung zu stellen, so dass zur Erkennung, ob ein Werkzeug (3) mit der Behandlungsvorrichtung (1) verbunden ist oder welches von mehreren Werkzeugen (3) mit der Behandlungsvorrichtung (1) verbunden ist, die zumindest eine Messspule (5, 5A) mit periodischer, insbesondere sinusförmiger, elektrischer Energie unterschiedlicher Frequenzen versorgbar ist.

13. Verfahren zur Erkennung ob ein Werkzeug (3) mit einer Behandlungsvorrichtung (1) nach einem der vorstehenden Ansprüche verbunden ist oder welches von mehreren Werkzeugen (3) mit der Behandlungsvorrichtung (1) verbunden ist, **dadurch gekennzeichnet, dass**
die zumindest eine Messspule (5, 5A) mit periodischer, insbesondere sinusförmiger, elektrischer Energie versorgt wird, aufgrund einer induktiven Kopplung zwischen dem in der Werkzeughalterung (22) aufgenommenen Werkzeug (3) und der mit der periodischen, insbesondere sinusförmigen, Energie versorgten zumindest einen Messspule (5, 5A) ein für jedes Werkzeug (3) spezifisches, durch die Auswertevorrichtung (4) dem jeweiligen Werkzeug (3) zuordenbares periodisches, insbesondere sinusförmiges, Messsignal generiert und von der Auswertevorrichtung (4) empfangen wird, und die Auswertevorrichtung (4) zur Erkennung, ob ein Werkzeug mit der Behandlungsvorrichtung (1) verbunden ist oder welches von mehreren Werkzeugen mit der Behandlungsvorrichtung (1) verbunden ist, die Phasenverschiebung der elektrischen Spannung und des elektrischen Stroms des periodischen, insbesondere sinusförmigen, Messsignals und vorzugsweise auch die Amplitude, insbesondere die Amplitudenextremwerte, der elektrischen Spannung des periodischen, insbesondere sinusförmigen, Messsignals bestimmt.

14. Verfahren nach Anspruch 13, **dadurch gekennzeichnet, dass**
in zumindest einem elektrisch leitfähigen Identifizierungselement (6) zumindest eines Werkzeugs (3) durch die induktive Kopplung zwischen diesem in der Werkzeughalterung (22) aufgenommenen Werkzeug (3) und der mit der periodischen, insbesondere sinusförmigen, Energie versorgten zumindest einen Messspule (5, 5A) ein elektrischer Wechselstrom und / oder elektrische Wirbelströme induziert wird / werden, der / die zur Generierung des dem jeweiligen Werkzeug (3) zuordenbaren periodischen, insbesondere sinusförmigen, Messsignals auf die in der zumindest einen Messspule (5, 5A) fließende periodische, insbesondere sinusförmige, elektrische Energie, insbesondere über Gegeninduktivität und / oder Ohmsche Verluste, rückwirkt / rückwirken.

15. Medizinische oder dentale Behandlungsvorrichtung (1) nach einem der vorstehenden Ansprüche 1 - 12, **gekennzeichnet durch**
ein medizinisches oder dentales Werkzeug (3), das umfasst: einen Werkzeugschaft (3B) und ein mit dem Werkzeugschaft (3B) verbundenes Arbeitsende (3A) zum Bearbeiten einer Behandlungsstelle, wobei der Werkzeugschaft (3B) zumindest ein elektrisch leitfähiges Identifizierungselement (6) aufweist, in dem durch eine induktive Kopplung zwischen dem Werkzeug (3) und der zumindest einen mit periodischer, insbesondere sinusförmiger, elektrischer Energie versorgten Messspule (5, 5A) ein elektrischer Wechselstrom und / oder elektrische Wirbelströme induzierbar ist / sind, der / die zur Generierung eines dem Werkzeug (3) zuordenbaren periodischen, insbesondere sinusförmigen, Messsignals auf die in der zumindest einen Messspule (5, 5A) fließende periodische, insbesondere sinusförmige, elektrische Energie, insbesondere über Gegeninduktivität und / oder Ohmsche Verluste, rückwirkt / rückwirken.

## Claims

1. A medical or dental treatment device (1), which can be connected to a plurality of different tools (3), wherein the medical or dental treatment device (1) comprises: a tool retainer (22) for connecting the treatment device (1) to a tool, at least one measuring coil (5, 5A) which is connected to an electrical energy supply device (12) via an electrical line (11) and which can be supplied with a periodic, in particular sinusoidal electrical energy supply from the electrical energy supply device (12), and an evaluation device (4) electrically connected to the at least one measuring coil (5, 5A) for detecting whether a tool is connected to the treatment device (1) or which of a plurality of tools is connected to the treatment device (1), wherein due to an inductive coupling between the tool (3) accommodated in the tool retainer (22) and the at least one measuring coil (5, 5A) supplied with the periodic electrical energy, a periodic, in particular sinusoidal measurement signal can be generated which is specific to each tool (3) and which can be assigned to the respective tool (3) by the evaluation device (4), **characterized in that**
the evaluation device (4) determines the phase shift of the electrical voltage and of the electric current of the periodic, in particular sinusoidal measurement signal for detecting whether a tool is connected to the treatment device (1) or which of a plurality of tools is connected to the treatment device (1).

2. The medical or dental treatment device (1) according to claim 1, **characterized in that**
the evaluation device (4) determines the amplitude, in particular the extreme values of the amplitude, of the electrical voltage of the periodic, in particular sinusoidal measurement signal for detecting whether a tool is connected to the treatment device (1) or which of a plurality of tools is connected to the treatment device (1).

3. The medical or dental treatment device (1) according to claim 1 or claim 2, having at least one tool (3) which can be connected to the treatment device (1), **characterized in that**
the at least one tool (3) has a tool shaft (3B) and a working end (3A) connected to the tool shaft (3B) for working on a treatment site, wherein the tool shaft (3B) comprises at least one electrically conductive identification element (6) in which through the inductive coupling between the tool (3) accommodated in the tool retainer (22) and the at least one measuring coil (5, 5A) supplied with the periodic, in particular sinusoidal energy, an alternating electric current and/or electrical eddy currents can be induced, which feed(s) back onto the periodic, in particular sinusoidal electrical energy flowing in the at least one measuring coil (5, 5A) in order to generate the periodic, in particular sinusoidal measurement signal which can be assigned to the respective tool (3), in particular via mutual inductance and/or ohmic losses.

4. The medical or dental treatment device (1) according to claim 3, **characterized in that**
the at least one electrically conductive identification element (6) is provided on a base material (7) of the tool shaft (3B), wherein the at least one electrically conductive identification element (6) and the base material (7) comprise different materials.

5. The medical or dental treatment device (1) according to claim 3 or claim 4, **characterized in that**
the at least one electrically conductive identification element (6) comprises a wire, a winding (13), a coil (14), a coating (15), a film (17), or a sleeve (16).

6. The medical or dental treatment device (1) according to one of claims 3, 4 or 5, **characterized in that**
the at least one electrically conductive identification element (6) forms a closed electrical circuit (37) surrounding the tool shaft (3B), in which the alternating electric current can flow, in particular about the tool shaft (3B) or about a longitudinal axis (38) of the tool shaft (3B).

7. The medical or dental treatment device (1) according to one of claims 3, 4 or 5, **characterized in that**
the at least one electrically conductive identification element (6) comprises two mutually separated ends (39A, 39B).

8. The medical or dental treatment device (1) according to one of claims 3 to 7, **characterized by** a plurality of different tools (3), each of which having a tool shaft (3B) with at least one electrically conductive identification element (6) wherein, in order to detect or differentiate the tools (3) by the evaluation device (4), the electrically conductive identification elements (6) of the plurality of tools (3) differ in at least one of the following parameters: the axial lengths of the electrically conductive identification element (6) with respect to a longitudinal axis (38) of the tool shaft (3B); the cross-sectional areas of the electrically conductive identification elements (6); the thicknesses of the electrically conductive identification elements (6) transversely to or radially to a longitudinal axis (38) of the tool shaft (3B); the thicknesses of the layers of the electrically conductive identification elements (6) deposited on the tool shafts (3B); the materials of the electrically conductive identification elements (6); the number of turns and/or the lengths of the electrically conductive identification elements (6) configured as coils (14) or windings (13); the wire diameter of the electrically conductive identification elements (6) configured as coils (14) or windings (13).

9. The medical or dental treatment device (1) according to claim 8, **characterized in that**
in order to detect or differentiate the plurality of different tools (3) by the evaluation device (4), the plurality of tools (3) differ in at least one of the following parameters: the base material (7) of the tool shaft (3B); the diameter of at least one section of the tool shaft (3B); the length of the tool shaft (3B).

10. The medical or dental treatment device (1) according to one of claims 3 to 9, **characterized in that**
the tool shaft (3B) of the at least one tool (3) comprises at least two electrically conductive and separated identification elements (6).

11. The medical or dental treatment device (1) according to one of the preceding claims, **characterized by**
a plurality of measuring coils (5, 5A) which are electrically connected to the evaluation device (4) for detecting whether a tool is connected to the treatment device (1) or which of a plurality of tools is connected to the treatment device (1).

12. The medical or dental treatment device (1) according to one of the preceding claims, **characterized by**
an electrical energy supply device (12) which is configured to provide the at least one measuring coil (5, 5A) with periodic, in particular sinusoidal electrical energy at different frequencies so that, in order to detect whether a tool (3) is connected to the treatment device (1) or which of a plurality of tools (3) is connected to the treatment device (1), the at least one measuring coil (5, 5A) can be supplied with periodic, in particular sinusoidal electrical energy at different frequencies.

13. A method for detecting whether a tool (3) is connected to a treatment device (1) according to one of the preceding claims, or which of a plurality of tools (3) is connected to the treatment device (1), **characterized in that**
the at least one measuring coil (5, 5A) is supplied with periodic, in particular sinusoidal electrical energy, due to an inductive coupling between the tool (3) accommodated in the tool retainer (22) and the at least one measuring coil (5, 5A) supplied with the periodic, in particular sinusoidal energy, a periodic, in particular sinusoidal measurement signal which is specific to each tool (3) and which can be assigned to the respective tool (3) by the evaluation device (4) is generated and is received by the evaluation device (4) and, in order to detect whether a tool is connected to the treatment device (1) or which of a plurality of tools is connected to the treatment device (1), the evaluation device (4) determines the phase shift of the electrical voltage and of the electric current of the periodic, in particular sinusoidal measurement signal and preferably also the amplitude, in particular the extreme values of the amplitude of the electrical voltage of the periodic, in particular sinusoidal measurement signal.

14. The method according to claim 13, **characterized in that**
in at least one electrically conductive identification element (6) of the at least one tool (3), due to the inductive coupling between said tool (3) accommodated in the tool retainer (22) and the at least one measuring coil (5, 5A) supplied with the periodic, in particular sinusoidal energy, an alternating electric current and/or electrical eddy currents is/are induced which feed(s) back onto the periodic, in particular sinusoidal electrical energy flowing in the at least one measuring coil (5, 5A), in particular via mutual inductance and/or ohmic losses, in order to generate the periodic, in particular sinusoidal measurement signal which can be assigned to the respective tool (3).

15. The medical or dental treatment device (1) according to one of the preceding claims 1 to 12, **characterized by**
a medical or dental tool (3) which comprises: a tool shaft (3B) and a working end (3A) connected to the tool shaft (3B) for working on a treatment site, wherein the tool shaft (3B) comprises at least one electrically conductive identification element (6) in which due to an inductive coupling between the tool (3) and the at least one measuring coil (5, 5A) supplied with periodic, in particular sinusoidal electrical energy, an alternating electric current and/or electrical eddy currents can be induced, which feed(s) back onto the periodic, in particular sinusoidal electrical energy flowing in the at least one measuring coil (5, 5A), in particular via mutual inductance and/or ohmic losses, in order to generate a periodic, in particular sinusoidal measurement signal which can be assigned to the tool (3).

## Revendications

1. Dispositif de traitement médical ou dentaire (1), lequel peut être raccordé à plusieurs outils (3) différents, dans lequel le dispositif de traitement médical ou dentaire (1) comprend: un porte-outil (22) pour raccorder le dispositif de traitement (1) à un outil, au moins une bobine de mesure (5, 5A) qui est raccordée via une ligne électrique (11) à un dispositif d'alimentation en énergie électrique (12) et peut être alimentée par le dispositif d'alimentation en énergie électrique (12) avec une énergie électrique périodique, en particulier sinusoïdale, et un dispositif d'évaluation (4) raccordé électriquement à l'au moins une bobine de mesure (5, 5A) pour reconnaître si un outil est raccordé au dispositif de traitement (1) ou lequel de plusieurs outils est raccordé au dispositif de traitement (1), dans lequel, du fait d'un couplage inductif entre l'outil (3) réceptionné dans le porte-outil (22) et l'au moins une bobine de mesure (5, 5A) alimentée par l'énergie électrique périodique, il est possible de générer un signal de mesure spécifique pour chaque outil (3), périodique, en particulier sinusoïdal, pouvant être attribué par le dispositif d'évaluation (4) à l'outil respectif (3), **caractérisé en ce que**
le dispositif d'évaluation (4), pour reconnaître si un outil est raccordé au dispositif de traitement (1) ou lequel de plusieurs outils est raccordé au dispositif de traitement (1), détermine le décalage de phase de la tension électrique et du courant électrique du signal de mesure périodique, en particulier sinusoïdal.

2. Dispositif de traitement médical ou dentaire (1) selon la revendication 1, **caractérisé en ce que** le dispositif d'évaluation (4), pour reconnaître si un outil est raccordé au dispositif de traitement (1) ou lequel de plusieurs outils est raccordé au dispositif de traitement (1), détermine l'amplitude, en particulier les valeurs extrêmes d'amplitude de la tension électrique du signal de mesure périodique, en particulier sinusoïdal.

3. Dispositif de traitement médical ou dentaire (1) selon la revendication 1 ou 2 avec au moins un outil (3) pouvant être raccordé au dispositif de traitement (1), **caractérisé en ce que**
l'au moins un outil (3) présente une queue d'outil (3B) et une extrémité de travail (3A) raccordée à la queue d'outil (3B) pour traiter un emplacement de traitement, dans lequel la queue d'outil (3B) présente au moins un élément d'identification électroconducteur (6) dans lequel, du fait du couplage inductif entre l'outil (3) réceptionné dans le porte-outil (22) et l'au moins une bobine de mesure (5, 5A) alimentée par l'énergie périodique, en particulier sinusoïdale, un courant électrique alternatif et/ou des courants de Foucault électriques peut/peuvent être induit(s), lequel/lesquels, pour la génération du signal de mesure périodique, en particulier sinusoïdal pouvant être attribué à l'outil (3) respectif, a/ont une répercussion sur l'énergie électrique périodique, en particulier sinusoïdale, circulant dans l'au moins une bobine de mesure (5, 5A), en particulier par l'intermédiaire d'une inductance mutuelle et/ou de pertes ohmiques.

4. Dispositif de traitement médical ou dentaire (1) selon la revendication 3, **caractérisé en ce que** l'au moins un élément d'identification électroconducteur (6) est prévu sur un matériau de base (7) de la queue d'outil (3B), dans lequel l'au moins un élément d'identification électroconducteur (6) et le matériau de base (7) présentent des matériaux différents.

5. Dispositif de traitement médical ou dentaire (1) selon la revendication 3 ou 4, **caractérisé en ce que**
l'au moins un élément d'identification électroconducteur (6) présente un fil, un enroulement (13), une bobine (14), un revêtement (15), un film (17), ou une gaine (16).

6. Dispositif de traitement médical ou dentaire (1) selon l'une des revendications 3, 4 ou 5, **caractérisé en ce que**
l'au moins un élément d'identification électroconducteur (6) forme un circuit électrique fermé (37) entourant la queue d'outil (3B), dans lequel le courant électrique alternatif peut circuler en particulier autour de la queue d'outil (3B) ou autour d'un axe longitudinal (38) de la queue d'outil (3B).

7. Dispositif de traitement médical ou dentaire (1) selon l'une des revendications 3, 4 ou 5, **caractérisé en ce que**
l'au moins un élément d'identification électroconducteur (6) présente deux extrémités (39A, 39B) séparées électriquement l'une de l'autre.

8. Dispositif de traitement médical ou dentaire (1) selon l'une des revendications 3-7, **caractérisé par**
plusieurs outils (3) différents, chacun d'entre eux présentant une queue d'outil (3B) avec au moins un élément d'identification électroconducteur (6), dans lequel, pour la reconnaissance ou la distinction des outils (3) par le dispositif d'évaluation (4), les éléments d'identification électroconducteurs (6) des plusieurs outils (3) diffèrent de par au moins l'un des paramètres suivants: les longueurs axiales des éléments d'identification électroconducteurs (6) par rapport à un axe longitudinal (38) de la queue d'outil (3B); les surfaces en section transversale des éléments d'identification électroconducteurs (6); les épaisseurs des éléments d'identification électroconducteurs (6) transversalement ou radialement à un axe longitudinal (38) de la queue d'outil (3B); les épaisseurs de couche des éléments d'identification électroconducteurs (6) déposés sur les queues d'outil (3B); les matériaux des éléments d'identification électroconducteurs (6); les nombres de spires et/ou longueurs des éléments d'identification électroconducteurs (6) réalisés en tant que bobines (14) ou enroulements (13); le diamètre de fil des éléments d'identification électroconducteurs (6) réalisés en tant que bobines (14) ou enroulements (13).

9. Dispositif de traitement médical ou dentaire (1) selon la revendication 8, **caractérisé en ce que**,
pour la reconnaissance ou distinction des plusieurs outils (3) différents par le dispositif d'évaluation (4), les plusieurs outils (3) diffèrent de par au moins l'un des paramètres suivants: le matériau de base (7) de la queue d'outil (3B); le diamètre d'au moins une partie de la queue d'outil (3B); la longueur de la queue d'outil (3B).

10. Dispositif de traitement médical ou dentaire (1) selon l'une des revendications 3 - 9, **caractérisé en ce que**
la queue d'outil (3B) de l'au moins un outil (3) présente au moins deux éléments d'identification électroconducteurs (6) et espacés l'un de l'autre.

11. Dispositif de traitement médical ou dentaire (1) selon l'une des revendications précédentes, **caractérisé par**
plusieurs bobines de mesure (5, 5A) raccordées électriquement au dispositif d'évaluation (4) pour reconnaître si un outil est raccordé au dispositif de traitement (1) ou lequel de plusieurs outils est raccordé au dispositif de traitement (1).

12. Dispositif de traitement médical ou dentaire (1) selon l'une des revendications précédentes, **caractérisé par**
un dispositif d'alimentation en énergie électrique (12) réalisé pour mettre à disposition, à l'au moins une bobine de mesure (5, 5A), de l'énergie électrique périodique, en particulier sinusoïdale, avec des fréquences différentes, de sorte que pour reconnaître si un outil (3) est raccordé au dispositif de traitement (1) ou lequel de plusieurs outils (3) est raccordé au dispositif de traitement (1), l'au moins une bobine de mesure (5, 5A) peut être alimentée en énergie électrique périodique, en particulier sinusoïdale, de fréquences différentes.

13. Procédé pour reconnaître si un outil (3) est raccordé à un dispositif de traitement (1) selon l'une des revendications précédentes ou lequel de plusieurs outils (3) est raccordé au dispositif de traitement (1), **caractérisé en ce que**
l'au moins une bobine de mesure (5, 5A) est alimentée en énergie électrique périodique, en particulier sinusoïdale, en raison d'un couplage inductif entre l'outil (3) réceptionné dans le porte-outil (22) et l'au moins une bobine de mesure (5, 5A) alimentée avec l'énergie périodique, en particulier sinusoïdale, un signal de mesure périodique, en particulier sinusoïdal, spécifique pour chaque outil (3) et pouvant être attribué par le dispositif d'évaluation (4) à l'outil respectif (3), est généré et est réceptionné par le dispositif d'évaluation (4), et le dispositif d'évaluation (4), pour reconnaître si un outil est raccordé au dispositif de traitement (1) ou lequel de plusieurs outils est raccordé au dispositif de traitement (1), détermine le décalage de phase de la tension électrique et du courant électrique du signal de mesure périodique, en particulier sinusoïdal, et de préférence également l'amplitude, en particulier les valeurs extrêmes d'amplitude de la tension électrique du signal de mesure périodique, en particulier sinusoïdal.

14. Procédé selon la revendication 13, **caractérisé en ce que**,
dans au moins un élément d'identification électroconducteur (6) d'au moins un outil (3), grâce au couplage inductif entre cet outil (3) réceptionné dans le porte-outil (22) et l'au moins une bobine de mesure (5, 5A) alimentée par l'énergie périodique, en particulier sinusoïdale, un courant électrique alternatif et/ou des courants de Foucault électriques est/sont induit(s), lequel/lesquels, pour la génération du signal de mesure périodique, en particulier sinusoïdal pouvant être attribué à l'outil (3) respectif, a/ont une répercussion sur l'énergie électrique périodique, en particulier sinusoïdale, circulant dans l'au moins une bobine de mesure (5, 5A), en particulier par l'intermédiaire d'une inductance mutuelle et/ou de pertes ohmiques.

15. Dispositif de traitement médical ou dentaire (1) selon l'une des revendications 1-12 précédentes, **caractérisé par**
un outil médical ou dentaire (3), lequel comprend: une queue d'outil (3B) et une extrémité de travail (3A) raccordée à la queue d'outil (3B) pour traiter un emplacement de traitement, dans lequel la queue d'outil (3B) présente au moins un élément d'identification électroconducteur (6) dans lequel, du fait d'un couplage inductif entre l'outil (3) et l'au moins une bobine de mesure (5, 5A) alimentée par l'énergie périodique, en particulier sinusoïdale, un courant électrique alternatif et/ou des courants de Foucault électriques peut/peuvent être induit(s), lequel/lesquels, pour la génération d'un signal de mesure périodique, en particulier sinusoïdal pouvant être attribué à l'outil (3) respectif, a/ont une répercussion sur l'énergie électrique périodique, en particulier sinusoïdale, circulant dans l'au moins une bobine de mesure (5, 5A), en particulier par l'intermédiaire d'une inductance mutuelle et/ou de pertes ohmiques.
